# EUROPEAN PATENT APPLICATION

(11) **EP 2 154 241 A2**
(43) Date of publication of application: **17.02.2010**
(21) Application number: 09010395.3
(22) Date of filing: 12.08.2009
(51) Int. Cl.: C12N 5/00, C12N 5/077, C12N 5/078, C12M 3/00, C12Q 1/02

(54) **Cell culture well-plates having inverted colloidal crystal scaffolds**

(30) Priority: 12.08.2008 US 228419
(71) Applicant: The Regents of the University of Michigan, Ann Arbor, MI 48104-2592 (US)
(72) Inventor: Kotov, Nicholas A., Ypsilanti, MI 48198 (US); Cortiella, Joaquin, Galveston, TX 77550 (US); Nichols, Joan E., Galveston, TX 77550 (US)
(74) Representative: Findeisen, Marco

(57) **Abstract**

An artificial bone marrow construct comprising a substrate having at least one well; a three dimensional biocompatible polymer matrix comprising a transparent polymer network containing microspherical voids, wherein the microspherical voids are each connected to at least one other void through inter-connecting pores; at least one LBL coating on a surface of at least one of the polymer network, voids and pores, a population of bone marrow cells comprising stem cells and stromal cells; and at least one bioactive agent. An artificial immune network comprising a polymer matrix with a population of immune cells comprising B-cells and T-cells is disclosed. Methods for testing the toxicity of drugs and other agents against bone marrow cells and methods for making universal blood using the artificial bone marrow constructs are also disclosed.

## Description

### CROSS-REFERENCE TO RELATED APPLICATIONS

This application is a continuation-in-part of United States Patent Application No. 11/656,362 filed on January 22, 2007. United States Patent Application No. 11/656,362 claims the benefit of U.S. Provisional Application No. 60/772,283, filed on February 10, 2006. The disclosures of the above applications are incorporated herein by reference.

### GOVERNMENT RIGHTS

This invention was made with government support under W81XWH-04-C-0139 awarded by ARMY/Medical Research and Medical Command (MRMC). The Government has certain rights in the invention.

### FIELD

The present disclosure relates to cell culture and, more particularly, relates to microplates having a three-dimension matrix scaffold.

### BACKGROUND

The statements in this section merely provide background information related to the present disclosure and may not constitute prior art.

The majority of cell culture studies are performed on two-dimensional (2-D) surfaces that include well microplates, tissue culture plates, tissue culture flasks, and Petri-dishes. In particular, cell culture microplates, which contain a large number of small identical wells for example 2, 4, 8, 16, 24, 48, 96, 384, and up to 1536 wells are used widely because they are ideal for the study of low numbers of cells and high throughput cellular assays. These plates are a standard in analytical research and clinical diagnostic testing. The disadvantage of conventional cell culture in microplates and flasks lies in the limitation to 2-D culture.

The importance of a three-dimensional (3-D) cell culture substrate has been demonstrated in some cellular adhesion, migration, proliferation, and differentiation studies because nearly all tissue cells *in-vivo* are embedded in a 3-D extracellular microenvironment with a complex and dynamic molecular composition. Artificial 2-D substrates are likely to misrepresent findings by forcing cells to adjust to flat and rigid surfaces unlike the *in-vivo* environment. For that reason, varying degrees of 3-D cell culture substrates, with properties between 2-D Petri dishes and *in-vivo* mouse models, have been developed with some bio- and synthetic-polymers.

Numerous studies have shown that a 3-D cell culture system offers a more realistic micro- and local-environment where the functional properties of cells can be observed and manipulated. However, there is no standard 3-D cell scaffold because of the variability of scaffolds resulting from existing scaffold-manufacturing techniques. Current scaffold-fabricating technologies, which can include porogen leaching, freeze-drying, and gas foaming, produce highly porous structures with stochastically arranged pores. The resultant scaffolds lack precision in the shape and dimension of pores and channels, surface chemistry, and mechanical properties, leaving the experimentalist without control over the 3-D cellular microenvironment. To obtain results that mimic the *in-vivo* cellular response and are highly reproducible, one requires a 3-D scaffold with precisely controlled properties. The present disclosure is a standard method for fabricating 3-D inverted colloidal crystal (ICC) scaffolds that fit directly into standard cell culture well plates, including 96-well microplates, with highly controllable macro-, micro- and nano-scale properties, minimizing product variability and experimental results. By making the ICC cell scaffold size fit to a cell culture well microplate, this new type of 3-D cell scaffolds can be easily accepted in the current research field.

Driven by the desire of the pharmaceutical industry to replace some portion of regular *in-vitro* drug testing and potentially some animal trials with experiments in 3-D cell cultures, which can reduce the cost and the time from inception to production of a new drug, much work is also being done on the development of replicas of *in-vivo* cellular structures.

Numerous materials and manufacturing processes have been tested to create 3-D scaffolds for 3-D *in-vitro* drug testing studies. However, many of them are impractical for mass drug testing due to strong light scattering/absorption and variability in the scaffold quality/topology. Virtually all of the commercially made 3-D scaffolds are made from ceramics or other inorganic materials and are difficult to incorporate in established drug evaluation protocols. Despite recent advances in tissue engineering and automation of biological systems, it would be useful to provide structures and automated methods of making biomimetic structures capable of growth and maintenance of cultured cells under controlled conditions to study the effects of biologically active molecules including hormones, growth and differentiation factors, cytokines, pharmaceuticals, enzymes, toxins, antigens and biological organisms.

Further areas of applicability will become apparent from the description provided herein. It should be understood that the description and specific examples are intended for purposes of illustration only and are not intended to limit the scope of the present disclosure.

### SUMMARY

The present disclosure provides an artificial bone marrow construct which serves and replicates a functional equivalent of bone marrow. The artificial bone marrow construct includes a substrate having at least one well and a three-dimensional biocompatible polymer matrix disposed in the at least one well. The polymer matrix comprises a transparent polymer network containing microspherical voids, the microspherical voids are each in communication with at least one other void through inter-connecting pores. The artificial bone marrow construct also has at least one LBL coating on a surface of at least one of the polymer network, voids and pores. The construct also has a population of bone marrow cells comprising stem cells and stromal cells and at least one bioactive agent.

The microspherical voids are each connected to at least one other void through inter-connecting pores having a certain diameter. At least a portion of the surface of the polymer network has at least one LBL coating for example a surface of a void and pores. The bone marrow construct sustains a population of bone marrow cells which can include, for example, stem cells and hematopoietic and non-hematopoietic stromal cells. The bone marrow construct also contains at least one bioactive agent.

In another aspect, the present disclosure provides an artificial immune network comprising a three dimensional biocompatible polymer matrix comprising a polymer network containing microspherical voids, wherein the microspherical voids are each connected to at least one other void through inter-connecting pores; a population of immune cells comprising B-cells and T-cells; and optionally at least one bioactive agent.

The present disclosure further relates to a method of making universal blood for use as a replacement of blood, for example, human blood, in transfusions and where donor blood is required, for example, as a result of trauma, surgery and disease. The method comprises: (a) culturing hematopoietic stem cells in the presence of a tissue culture medium comprising a first biological active agent in an amount sufficient to induce the differentiation of hematopoietic stem cells into red blood cell progenitor cells in an artificial bone marrow construct; (b) adding a second biological active agent to the artificial bone marrow construct, the second biological active agent is in an amount sufficient to increase the numbers of the red blood cell progenitor cells in the artificial bone marrow construct; (c) adding a third biological active agent to the red blood cell progenitor cells to differentiate the red blood cell progenitor cells to form mature red blood cells; (d) collecting the mature red blood cells from the artificial bone marrow construct aseptically and (e) storing the mature red blood cells in a sterile container for use. The universal red blood cells can be treated to remove surface antigens such as the ABO RBC antigens and/or the Rhesus factor prior to administration as a universal blood product.

Further areas of applicability will become apparent from the description provided herein. It should be understood that the description and specific examples are intended for purposes of illustration only and are not intended to limit the scope of the present disclosure.

### DRAWINGS

The drawings described herein are for illustration purposes only and are not intended to limit the scope of the present disclosure in any way.

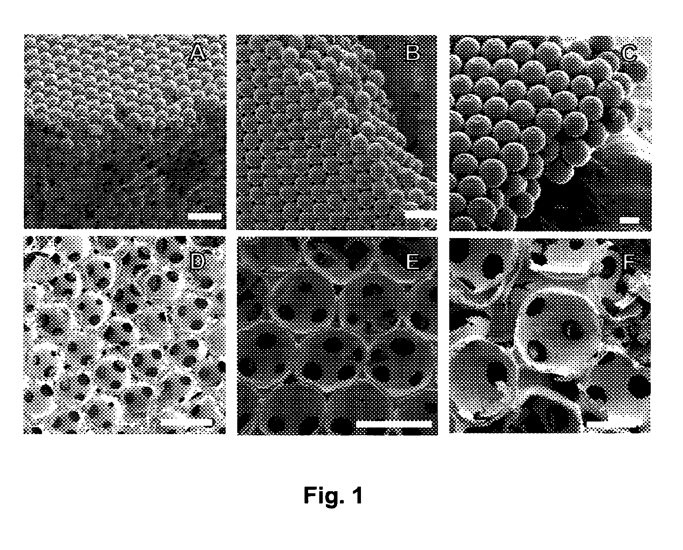

FIGs. 1A - 1F show scanning electron micrographs of templated micropsheres layered in a highly ordered array. FIGs. 1A - 1C show scanning electron micrographs of the microspheres formed in a solid colloidal array geometry. FIGs. 1D -1F show scanning electron micrographs of the inverted colloidal crystal scaffolds after the micropsheres have been removed revealing the three dimensional polymer network wherein the voids or cavities shown in cross-section are interconnected with pores. FIGs. 1E and 1F are higher magnification electron micrographs of FIG. 1D.

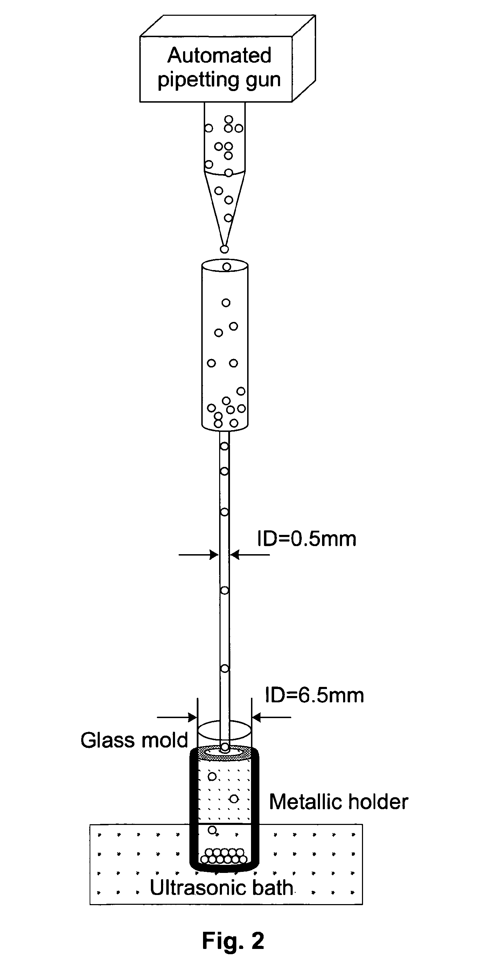

FIG. 2 is an illustration of an automated method for forming the highly ordered solid colloidal array in accordance with the embodiments of the present disclosure.

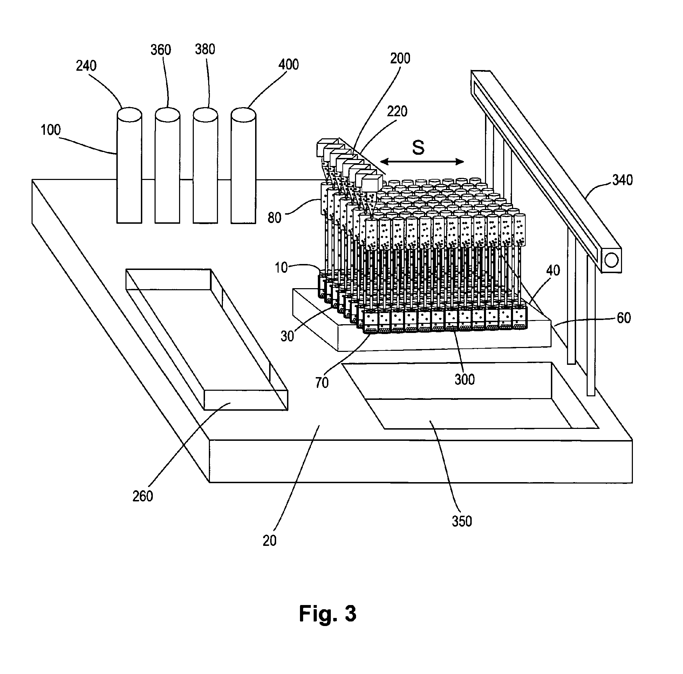

FIG. 3 is a perspective view of an automated apparatus for the fabrication of inverted colloidal crystal scaffolds in accordance with the present disclosure.

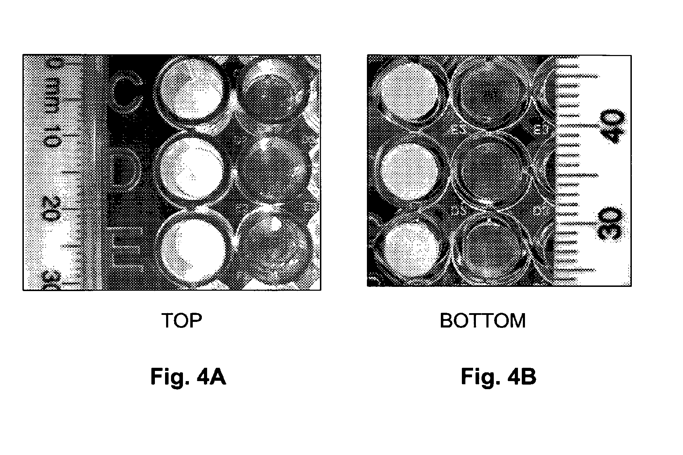

FIGs. 4A and 4B are photographs of the ICC colloidal crystal scaffold layered in some wells of a 96-well microplate substrate. FIG. 4A shows a photograph taken from the top of the microplate. FIG. 4B shows a photograph depicting the bottom of the 96-well microplate.

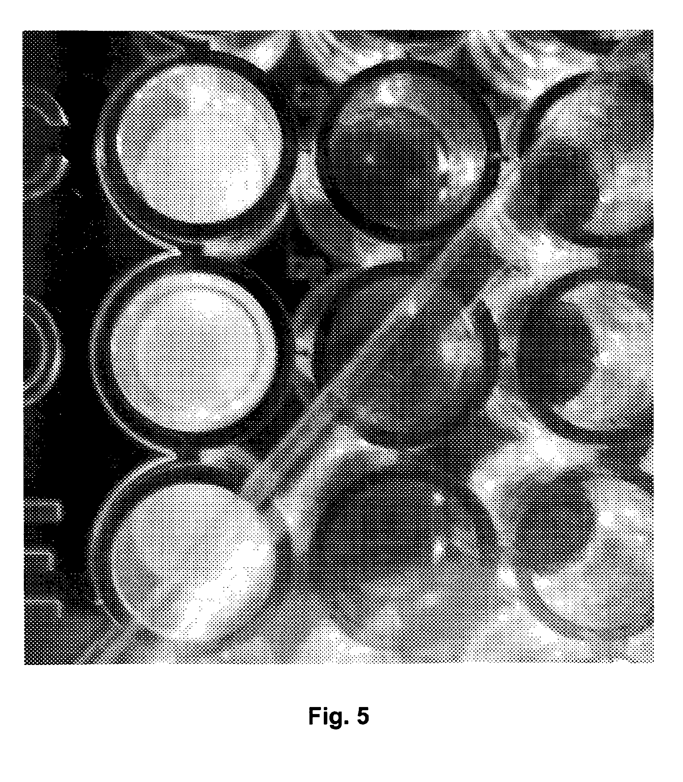

FIG. 5 is a photograph of 96-well microplate containing ICC scaffolds of the present disclosure after covering with a transparent sealing tape.

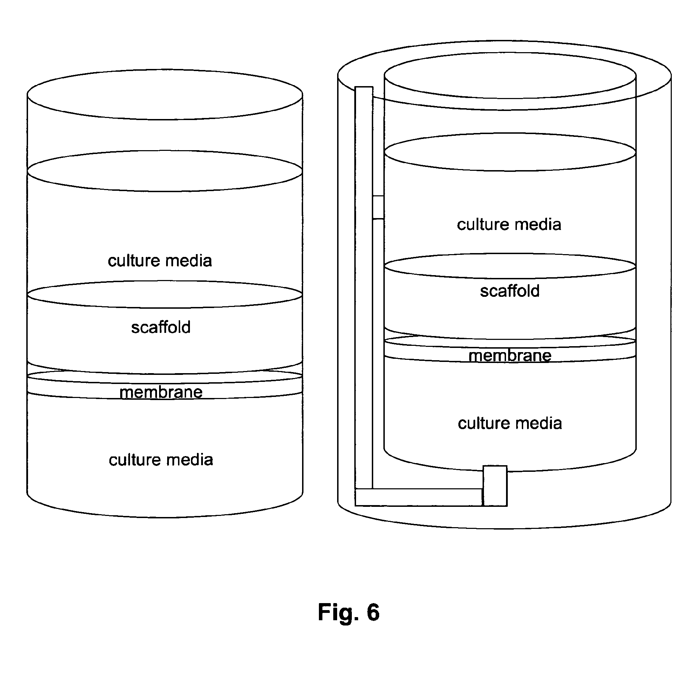

FIG. 6 is an illustration of a substrate arrangement containing an ICC scaffold layered on a membrane with culture media and a second substrate arrangement illustrating recirculating channels to circulate media throughout the scaffold.

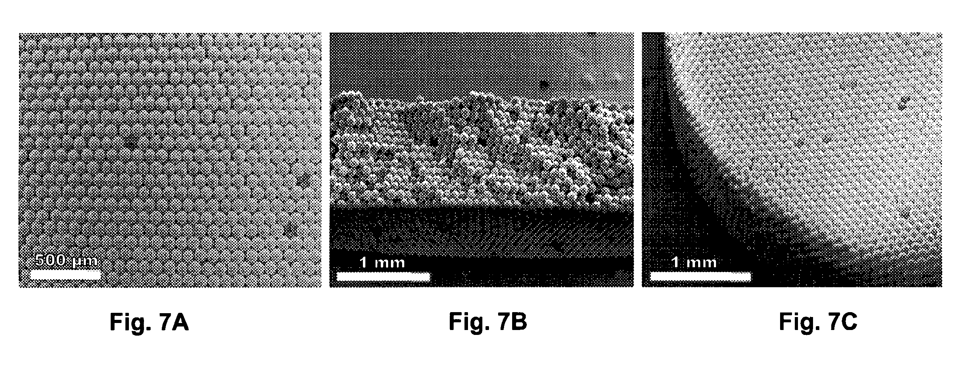

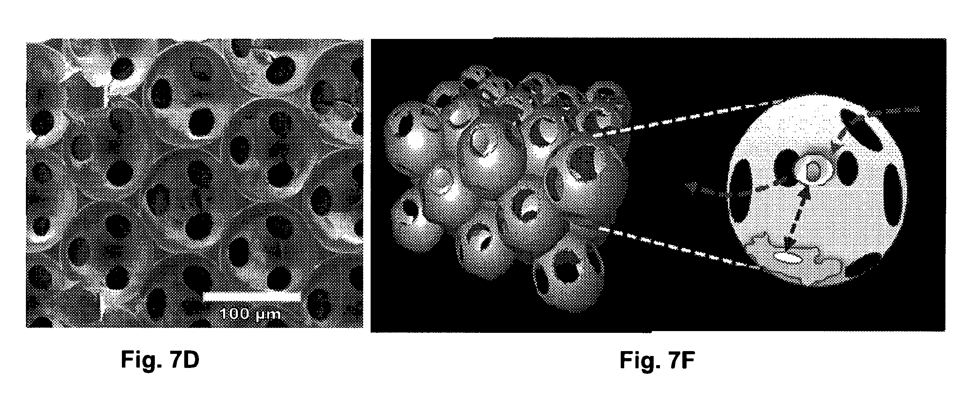

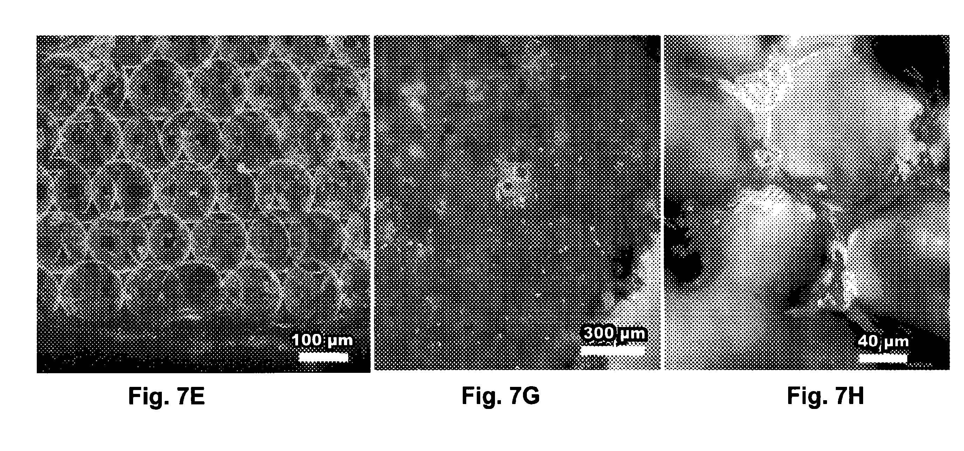

FIG. 7 shows the ICC scaffolds under scanning electron microscopy revealing the structural features of the scaffold and the colonization of cells in and around the voids of the scaffolds. FIGs. 7A - 7C show scanning electron microscopy micrographs of a colloidal crystal array made from 110µ polystyrene beads in different orientations. FIG. 7A depicts a photomicrograph of the general planar orientation of the colloidal crystals made in accordance with the present disclosure. FIG. 7B depicts a scanning electron photomicrograph of the cross-section of the typical colloidal crystals used here. FIG. 7D depicts a scanning electron microscopy of the ICC scaffolds from silicate gel. FIG. 7E depicts a scanning photomicrograph of a 3-D reconstructed poly (acrylamide) hydrogel ICC scaffold after 5 bilayers of FITC-albumin and PDDA coating using the LBL method described in accordance with the present disclosure. FIG. 7F is a rendition of the 3-D schematics of inverted colloidal crystals and cell contacts within. FIGs. 7G and 7H depict photomicrographs of ICC scaffolds after 5 days in culture with HS-5 bone marrow stromal cells in accordance with the present disclosure. Prior to seeding onto the scaffolds, the HS-5 cells were tagged with CFSE, a cell dye that acquires green fluorescence in live cells. (Scale bar D=100µm).

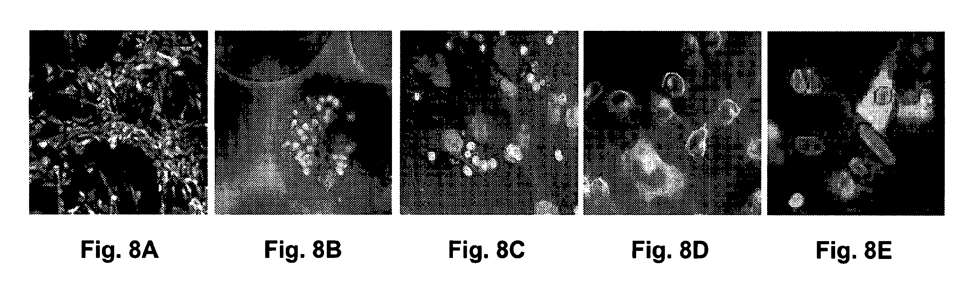

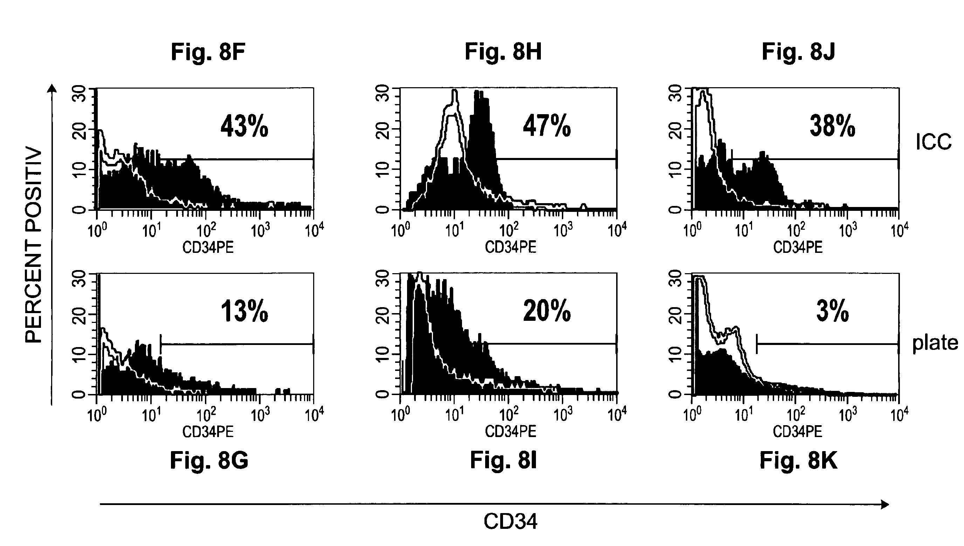

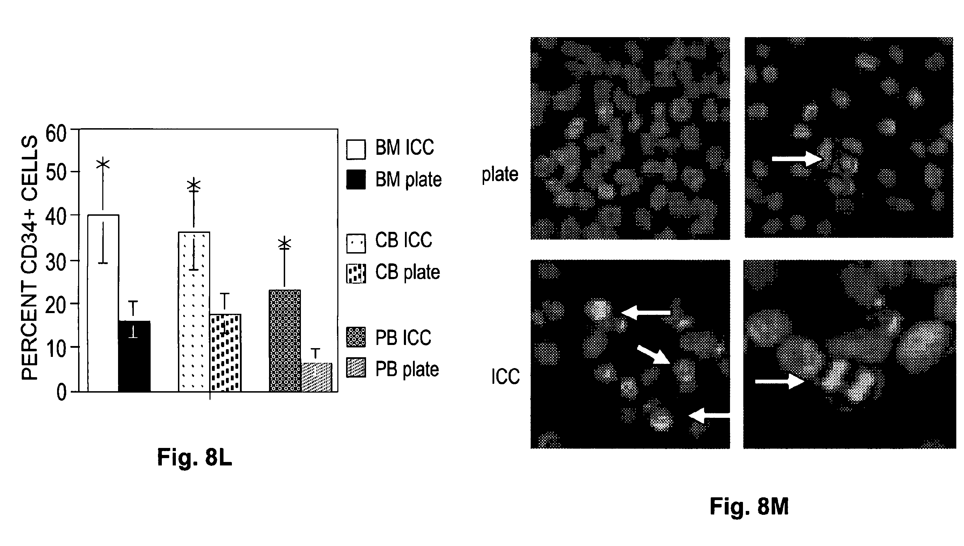

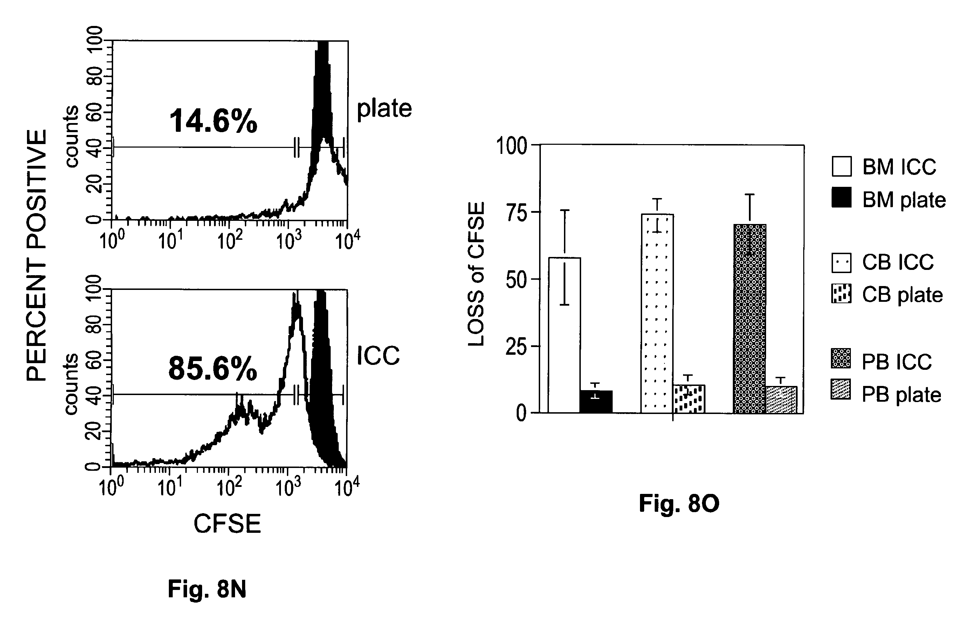

FIG. 8 shows the survival and growth of CD34+ and stromal cells in the ICC scaffold after 3-28 days. FIGs. 8A - 8E show confocal microscopy images of 7 µm frozen sections of hydrogel ICC scaffolds. FIG. 8A shows stromal cells cultured for 3 days were stained with CD105a stromal cell marker for visualization of the developing stromal cell network (green), 200X. FIG. 8B shows a confocal micrograph image of CD34+ HSCs seeded onto the ICC scaffold and imaged after 1 one day of stromal cell culture and imaged at a magnification of 400X. FIG. 8C shows a confocal micrograph image of ICC scaffold cultures on day 14 in the culture. Stromal cells were stained with anti-CD105a antibodies and CD34+ cells were separately stained using anti-CD34 antibodies and imaged at a magnification of 630X. FIG. 8D shows a confocal image of sections of 28 day ICC HSC cultures stained for actin and CD34 and imaged at a magnification of 630X. FIG. 8E shows the same cultures as in FIG. 8D stained for CD150. Nuclear material was stained with DAPI nuclear stain. FIGs. 8F, 8H and 8J show flow cytometry histograms depicting relative numbers of cells staining positive for CD34 derived from 3-D ICC scaffolds. FIGs. 8G, 8I and 8K show flow cytometry histograms depicting relative numbers of cells staining positive for CD34 positive cells derived from 2D plate cultures. The cells used in FIGs. 8F and 8G were obtained from bone marrow. The cells used in FIGs. 8H and 8I were obtained from cord blood. The cells used in FIGs. 8J and 8K were obtained from peripheral blood. FIG. 8L shows graphically the relative numbers of CD34+ cells on day 28. Significantly more CD34+ cells were seen in ICC cultures for bone marrow (BM) (P=0.01), cord blood (CB) (P=0.004), or peripheral blood (PB) (P=0.03) than for donor matched 2D plate culture in a total of six experiments. FIG. 8M shows confocal microscopy images of 28 day 2D plate culture (8M, top imaged at 400x) and 3-D ICC cultured cells (FIG. 8M, bottom, left imaged at 400x, and right imaged at 630x) stromal cell peripheral blood derived CD34+ cell seeded cultures. Plate and ICC cultures were stained for cell surface expression of CD34 and DAPI nuclear stain. There are fewer CD34+ cells in the plate (top micrographs) as compared to the ICC (bottom micrographs) cultures. Numerous mitotic figures indicate proliferation of CD34+ cells (white arrows) were seen in 28 day ICC cultures but not in plate cultures. FIG. 8N top panel (plate) depicts flow cytometric histograms of CFSE levels in cord blood derived CD34+ HSCs for donor matched 2D plate cultures and in bottom panel (ICC) ICC scaffold cultures. FIG. 8O shows graphically, the relative HCS proliferation by CFSE loss for ICC and 2D plate cell cultures for CD34+ cell sources indicated (averages for 5 experiments).

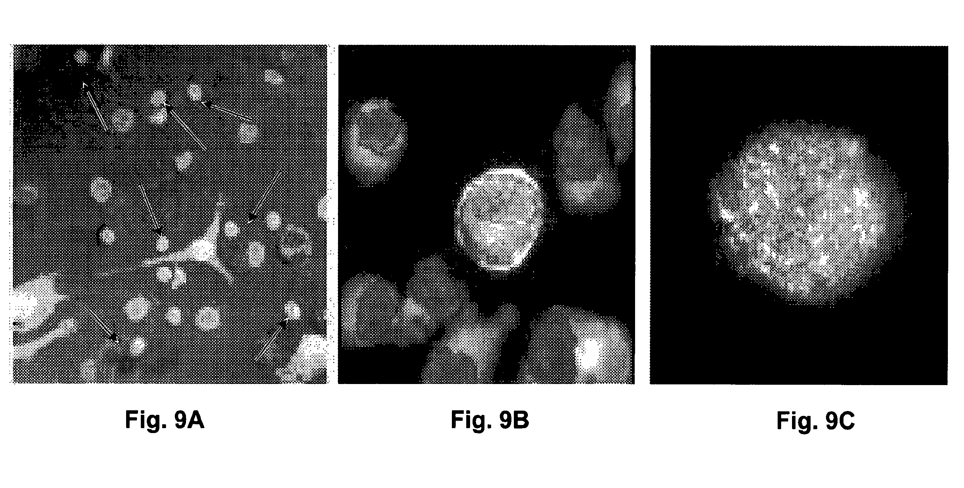

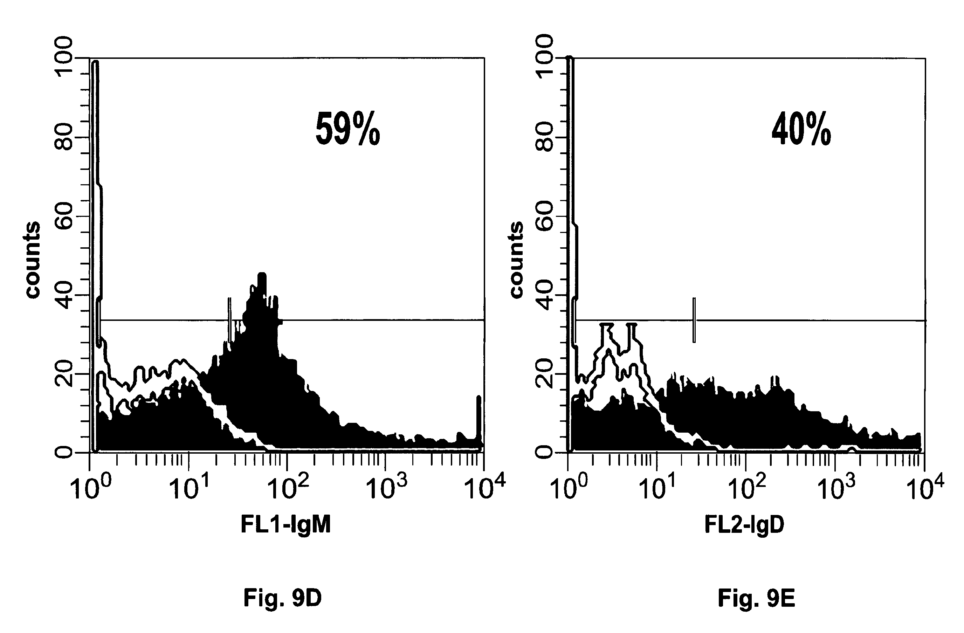

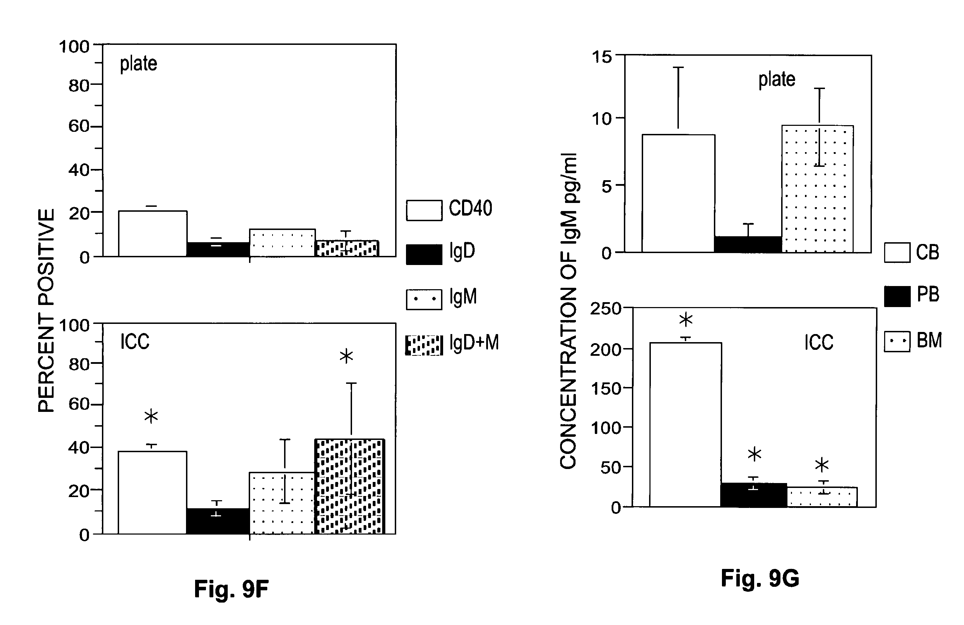

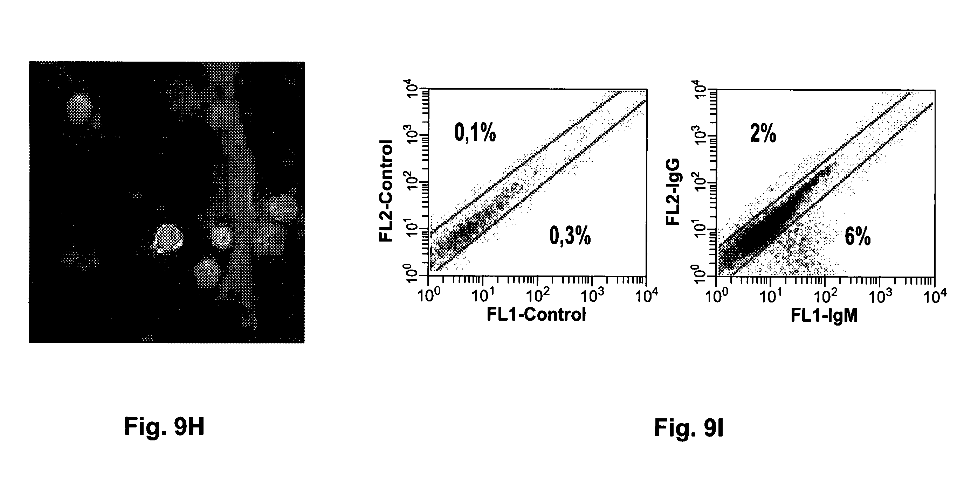

FIG. 9 shows CD34+ HSCs from cord blood cultured for 28 days in 2D or ICC scaffold cultures. FIGs. 9A - 9C show confocal microscopy images of 7µm sections hydrogel scaffolds. FIG. 9A shows a confocal micrograph image of nuclear RAG-1 expression and surface expression of IgM, at day 14. Arrows points to RAG-1 positive nuclei. FIG. 9B shows a confocal micrograph of cell surface co-expression of CD19 and IgM at day 28. FIG. 9C shows a confocal micrograph of co-expression of cell surface IgM and IgD at day 28. FIGs. 9D - 9E show flow cytometry histograms representing relative numbers of cells undergoing CD34+ differentiation. FIG. 9D shows a flow cytometry histogram showing expression of IgM. FIG. 9E shows a flow cytometry histogram showing expression of IgD. FIG. 9F shows a graph illustrating the average expression of CD40, IgM, IgD and IgM+IgD co-expression for plate and ICC cultures using CD34+ from cord blood. FIG. 9G is a graphical illustration of the comparison of IgM expression for plate and ICC cultures for different CD34+ sources. FIG. 9H shows a confocal micrograph of 7 µm section of ICC culture stained for expression of IgG and CD105 (a stromal cell marker). FIG. 9I depicts a left panel showing relative numbers of cells using flow cytometry data for IgG versus IgM expression and class switch for cord blood CD34+ HSC cells in an isotype control and the right panel showing relative numbers of cells showing IgG versus IgM expression and class switch for cord blood CD34+ HSC cells after exposure to influenza A/Caledonia virus.

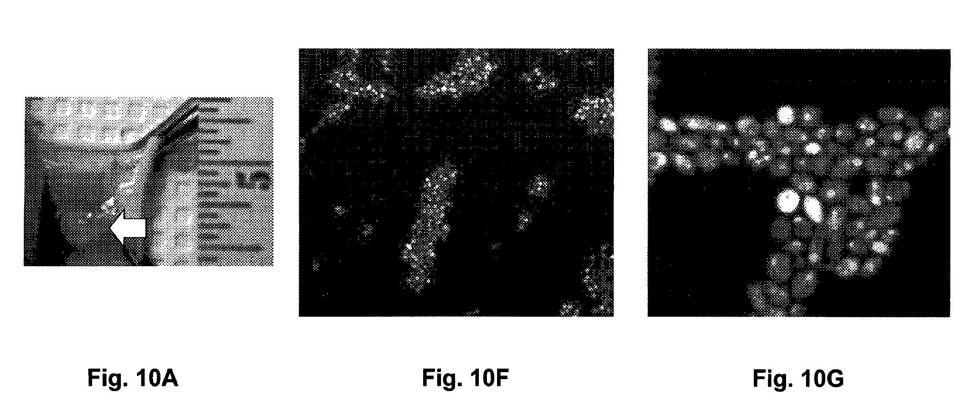

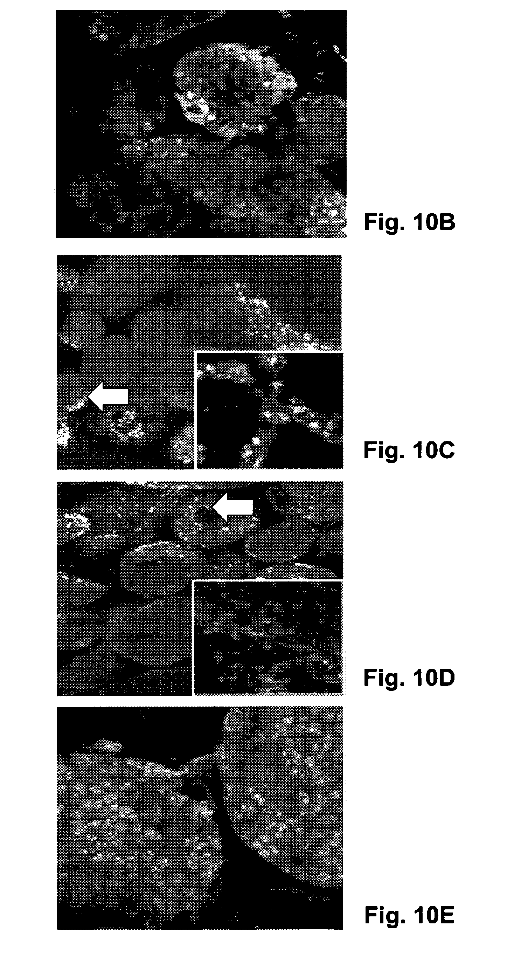

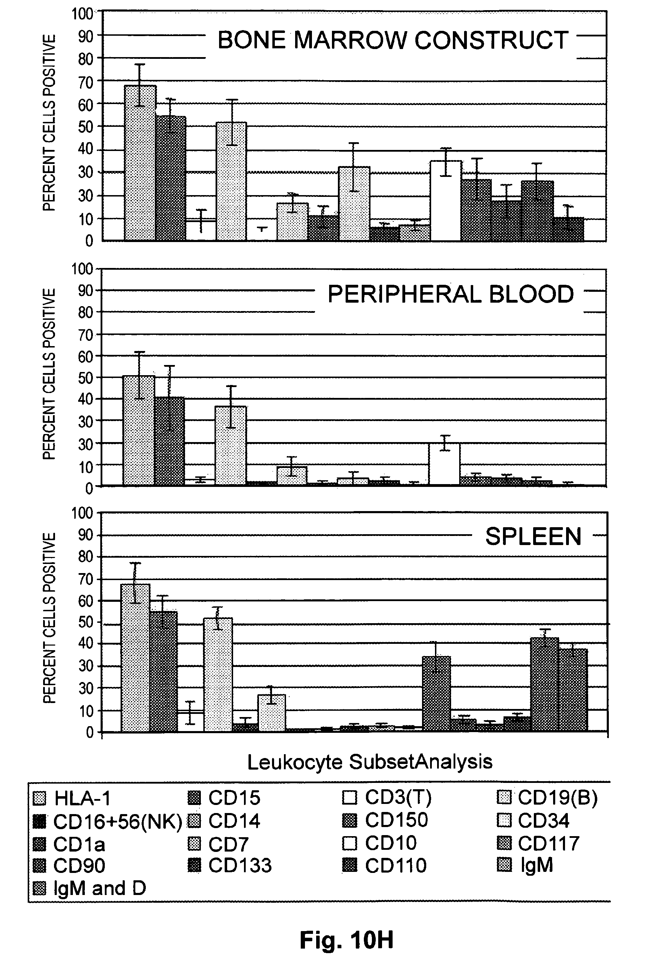

FIG. 10 shows the *in-vivo* implantation of the ICC scaffold and the evaluation of bone marrow construct, bone marrow derived cells, peripheral blood and spleen cells after about 3 days to about 14 days implantation of hydrogel ICC scaffolds on the backs of eight SCID mice. The scaffolds were seeded with CFSE labeled cord blood derived CD34+ HSCs and cultured for about 3 to about 7 days before implantation. FIG. 10A depicts a photograph illustrating a high degree of vascularization seen in the regions near the site of the implanted ICC construct *in-vivo.* FIGs. 10B - 10G depict confocal microscopy images of 7 µm frozen sections of the ICC-bone marrow construct using DAPI and CFSE. FIG. 10B depicts a photomicrograph of Human MHC-Class I staining, at 400X magnification. FIG. 10C depicts a photomicrograph of cells stained for CD34. The insert in FIG. 10C depicts the stained cells imaged at 630X magnification. FIG. 10D depicts a photomicrograph of cells stained for CD150. The insert in FIG. 10D depicts the stained cells imaged at 630X magnification. FIG. 10E depicts a photomicrograph of cells stained for CD133. FIG. 10F depicts a photomicrograph of cells stained for CD19 and FIG. 10G depicts a photomicrograph of cells stained for IgM expression imaged at 630X magnification. FIG. 10H is a graphical representation of flow cytometry evaluation of cell phenotypes found in the bone marrow construct, peripheral blood and spleens of SCID mice receiving constructs. For all flow cytometry data 10,000 events were collected for each sample. Isotype control staining was less than 2% cells positive for all antibodies used.

### DETAILED DESCRIPTION

The following description is merely exemplary in nature and is not intended to limit the present disclosure, application, or uses. In accordance with the present disclosure, it has been found that inverted colloidal crystal (ICC) scaffolds comprising a biocompatible three-dimensional matrix can be made and used to sustain and promote the growth and differentiation of living cells conveniently produced in tissue culture plates, including microplates and *in-vivo.* The present disclosure described herein further describes methods for the automated system of fabricating ICC scaffolds for standard cell culture plates, including without limitation, microplate tissue culture plates for use in assays relating to: cell-biology, toxicology, pharmacology, biochemistry, molecular biology, immunology and pathology.

In some embodiments, autologous, allogeneic and xenogeneic cells can be seeded, grown and manipulated in the ICC scaffolds using established cell-biology protocols commonly known in the art. The ICC scaffolds can be designed to advance current biological fields, including cell biology, biochemistry, molecular biology, microbiology, and systems biology. For example, cell culture well microplates are commonly used in stem cell biology studies to perform multiple experiments using a limited number of stem cells. Additionally, research has shown that a 3-D culture environment can significantly reduce or eliminate the use of expensive cytokines that are necessary in 2-D stem cell cultures. Because the differentiation of stem cells can be highly influenced by signals from the 3-D environment, a uniform and highly controlled 3-D substrate within each well on the cell culture well microplate will improve economically current stem cell research techniques.

### ICC Scaffolds

In some embodiments, the three dimensional inverted colloidal crystal scaffold comprises a substrate having at least one well and a three dimensional polymer matrix comprising a transparent polymer network having a plurality of empty spherical cavities having interconnected pores arranged in a hexagonal crystal lattice See FIGs. 1A - 1C. As shown in FIG.1, the ICC scaffolds comprise a transparent 3-D polymer matrix containing a porosity consisting of voids or cavities having one or more interconnected pores between adjacent voids. In some embodiments, the voids are seeded with cells to form a transparent polymer ICC cell scaffold. As used herein, the 3-D polymer matrix can comprise any transparent, biocompatible polymer including for example, polystyrene, collagen gel, fibrin gel, poly(lactic acid), polypeptides, as well as co-polymers of these compounds, hydrogels, bioglasses or inorganic gels. The ICC scaffold can be placed in any substrate including without limitation, any suitable tissue culture plate having at least one well with at least one generally planar surface. In some embodiments, the substrate is a microplate having 48, 96, 384 or 1536 wells. In some embodiments, ICC scaffolds can be manufactured in cell culture plates having a plurality of wells ranging from 2 to 1536 identical or different sized wells. In some embodiments described herein, the ICC scaffolds can be manufactured and utilized to fit the wells of a cell culture well microplate (e.g. 24, 48, 96 384, or 1536 wells) to improve and standardize the cell growth environment of existing experiments, without significantly altering the procedures and materials required by the scientist.

In some embodiments, the ICC scaffold comprises a cell culture plate having at least one well comprising a planar surface disposed within the well. Generally, the substrate can be any commonly used cell-culture material that is inert and biocompatible, for example plastics, glass, ceramic, metallic and combinations thereof. In non-limiting examples, the substrate containing wells within for example, of the microplates, can comprise polypropylene, polyethylene terephthalate, polytetrafluoroethylene, polyaryletherketone, nylon, fluorinated ethylene propylene, polybutylester, silicone or combinations thereof. In some embodiments, cell culture plates and wells therein, can include for example, one well cell culture plate (square or round Petri dish), 2 well cell culture dish, 4 well cell culture dish, 8 well cell culture dish, 12 well cell culture dish, 24 well cell culture dish, 48 well cell culture dish, 96 well cell culture microplate, 384 well cell culture microplate and 1536 well microplate. The cell culture plates, dishes, or microplates can be made of polypropylene, polycarbonate, polystyrene and other commonly known tissue culture plastic. In some embodiments, the cell culture plate has a flat-bottomed well, meaning that the surface upon which the ICC scaffold is made contains a substantially planar surface having a wall generally made of the same material orthogonal to the plane of the surface capable of containing a predetermined volume of liquid containing a hexagonal array of microspheres.

In some embodiments, the ICC scaffolds can comprise polymers that are biocompatible including polymers that impart both high transparency and elasticity. In some embodiments, the polymer can be a hydrogel. Hydrogels may be formed from covalently or non-covalently crosslinked materials, and may be nondegradable ("biostable") in a physiological environment or broken down by natural processes, referred to as biodegradable or bioabsorbable. The hydrogels generally exclude silica or metallic polymer matrices. The breakdown process may be due to one of many factors in the physiological environment, such as enzymatic activity, heat, hydrolysis, or others, including a combination of these factors.

Hydrogels can be crosslinked by variety of linkages, which may be reversible or irreversible. Reversible linkages can be due to ionic interaction, hydrogen or dipole type interactions or the presence of covalent bonds. Covalent linkages for absorbable or degradable hydrogels can be chosen from any of a variety of linkages that are known to be unstable in an animal physiological environment due to the presence of bonds that break either by hydrolysis (e.g., as found in synthetic absorbable sutures), enzymatically degraded (e.g., as found in collagen or glycosamino glycans or carbohydrates), or those that are thermally labile (e.g., azo or peroxy linkages).

In some embodiments, the hydrogel materials appropriate for use in the present disclosure should be physiologically acceptable and should be swollen in the presence of liquid, including water, saline and tissue culture media. The hydrogel can be formed by polymerization of monomer precursor solution in the well of the substrate.

In some embodiments, hydrogels can be formed from natural, synthetic, or biosynthetic polymers. Natural polymers can include glycosminoglycans, polysaccharides, proteins and the like. In some embodiments, illustrative examples of glycosaminoglycans can include dermatan sulfate, hyaluronic acid, the chondroitin sulfates, chitin, alginate heparin, keratan sulfate, keratosulfate, and derivatives thereof.

In general, the glycosaminoglycans can be extracted from a natural source and purified and derivatized. However, they also may be synthetically produced or synthesized by modified microorganisms such as bacteria. These materials may be modified synthetically from a naturally soluble state to a partially soluble or water swellable or hydrogel state. This modification can be accomplished by well-known techniques, such as by conjugation or replacement of ionizable or hydrogen bondable functional groups such as carboxyl and/or hydroxyl or amine group with other more hydrophobic groups.

The polymerizable hydrogels are made by polymerizing either through photo-curing, actinic radiation (UV, ion-beam and other ionizing radiation), or by cross-linking hydrogel monomers (including chemical, enzymatic and glycation). Hydrogels can be polymers, homopolymers, heteropolymers, co-polymers and block co-polymers. Suitable hydrogels can include, but are not limited to, aminodextran, dextran, DEAE-dextran, chondroitin sulfate, dermatan, heparan, heparin, chitosan, polyethyleneimine, polylysine, dermatan sulfate, heparan sulfate, alginic acid, pectin, carboxymethylcellulose, hyaluronic acid, agarose, carrageenan, starch, polyvinyl alcohol, cellulose, polyacrylic acid, poly(meth) acrylates, poly meth(methacrylate) PMMA, polyacrylamide, polyhydroxyalkanoates (PHA and PHB), polycaprolactone, polyetheretherketone, polyglycolide, poly-3-hydroxybutyrate, polyethylene glycol, or the salt or ester thereof, or a mixture thereof.

Synthetic polymeric hydrogels generally swell or expand to a very high degree, usually exhibiting a 2 to 100-fold volume increase upon hydration from a substantially dry or dehydrated state. Synthetic hydrogels may be biostable or biodegradable or bioabsorbable. Biostable hydrophilic polymeric materials that form hydrogels useful for practicing the present disclosure include poly(hydroxyalkyl methacrylate) including poly(meth) methacrylates, poly(electrolyte complexes), poly(vinylacetate) cross-linked with hydrolysable bonds, and water-swellable N-vinyl lactams. The swellable hydrogel can be used in manufacturing of the well-containing scaffolds by placing unswelled state of the hydrogel into the scaffold and transferring it to the swollen state to fit tightly in the well. In some embodiments, swellable hydrogels can be used for cell extraction from the scaffolds. The scaffold with attached cells can be placed in a media inducing swelling and the expansion of the hydrogel causing the detachment and release of the cells into the media.

Other suitable hydrogels can include hydrophilic hydrogels known as CARBOPOL®, a registered trademark of B. F. Goodrich Co., Akron, Ohio, for acidic carboxy polymer (Carbomer resins are high molecular weight, allylpentaerythritol-crosslinked, acrylic acid-based polymers, modified with C10-C30 alkyl acrylates), polyacrylamides, such as those marketed under CYANAMER®, a registered trademark of Cytec Technology Corp., Wilmington, DE, USA, polyacrylic acid marketed under GOOD-RITE®, a registered trademark of B. F. Goodrich Co., Akron, Ohio, polyethylene oxide, starch graft copolymers, acrylate polymer marketed under AQUAKEEP®, a registered trademark of Sumitomo Seika Chemicals Co., Japan, ester crosslinked polyglucan, and the like. Such hydrogels are described, for example, in U.S. Pat. No. 3,640,741 to Etes, U.S. Pat. No. 3,865,108 to Hartop, U.S. Pat. No. 3,992,562 to Denzinger et al., U.S. Pat. No. 4,002,173 to Manning et al., U.S. Pat. No. 4,014,335 to Arnold and U.S. Pat. No. 4,207,893 to Michaels and in the Handbook of Common Polymers, (Scott & Roff, Eds.) Chemical Rubber Company, Cleveland, Ohio.

Hydrogels can also be formed to be responsive to changes in environmental factors, such as pH, temperature, ionic strength, charge, etc., by exhibiting a corresponding change in physical size or shape, so-called "smart" gels. For example, thermoreversible hydrogels, such as those formed of amorphous N-substituted acrylamides in water, undergo reversible gelation when heated or cooled about certain temperatures (lower critical solution temperature, LCST). Prevailing gel formation mechanisms include molecular clustering of amorphous polymers and selective crystallization of mixed phases of crystalline materials. Such gels, which are insoluble under physiological conditions, also advantageously can be used for practicing the present disclosure.

It is also possible to affect the rate at which a substantially dehydrated hydrogel rehydrates in a physiological environment, such as encountered upon implantation in an animal. For example, creating a porous structure within the hydrogel by incorporating a blowing agent during the formation of the hydrogel may lead to more rapid re-hydration due to the enhanced surface area available for the water front to diffuse into the hydrogel structure.

The hydrogel forming precursors for the foregoing ICC scaffolds can be selected so that, for example, a free radical polymerization is initiated when two components of a redox initiating system are brought together.

In addition, the driving force for water to penetrate a dehydrated hydrogel also may be influenced by making the hydrogel hyperosmotic relative to the surrounding physiological fluids. Incorporation of charged species within hydrogels, for example, is known to greatly enhance the swellability of hydrogels. Thus the presence of carboxyl or sulfonic acid groups along polymeric chains within the hydrogel structure may be used to enhance both degree and rate of hydration. The surface to volume ratio of the implanted hydrogels also is expected to have an impact on the rate of swelling. For example, crushed dried hydrogel beads are expected to swell faster to the equilibrium water content state than a rod shaped implant of comparable volume.

Any of a variety of techniques may be used to form hydrogels in the cell culture plate or microplate. For example, monomers or macromers of hydrogel forming compositions can be further polymerized to form three dimensionally cross-linked hydrogels. The crosslinking may be covalent, ionic, and or physical in nature. Polymerization mechanisms permitting *in-situ* formation of hydrogels are per se known, and include, without limitation, free radical, condensation, anionic, or cationic polymerizations. The hydrogels also may be formed by reactions between nucleophilic and electrophilic functional groups, present on one or more polymeric species, which are added either simultaneously or sequentially. The formation of hydrogels also may be facilitated using external energy sources, such as in photoactivation, by UV light, thermal activation and chemical activation techniques.

Polymer precursors used to make the ICC scaffold, including hydrogels, can be fluorecscently labeled during the polymer synthesis or after polymerization to facilitate imaging processing of the cells contained in the ICC scaffolds. The fluorescent labeling can involve addition of specific dyes to the hydrogel composition or specific fluorescent groups to the monomer(s) in the polymerization process. The dyes can be covalently, ionically, cooperatively, hydrophobically or otherwise bonded, for instance using hydrogen, donor-acceptor, van-der Waals, bonds, to the hydrogel matrix.

Synthesis and biomedical and pharmaceutical applications of absorbable or biodegradable hydrogels based on covalently crosslinked networks comprising polypeptide or polyester components as the enzymatically or hydrolytically labile components, respectively, are known in the art. In some embodiments, the hydrogels useful in the present disclosure can be made of water-soluble polymers, such as polyvinyl pyrrolidone, which have been crosslinked with naturally derived biodegradable components such as those based on albumin.

Totally synthetic hydrogels have been studied for controlled drug release and membranes for the treatment of post-surgical adhesion and are contemplated for use in the present disclosure. Those hydrogels are based on covalent networks formed by the addition polymerization of acrylic-terminated, water-soluble polymers that have at least one biodegradable spacer group separating the water soluble segments from the crosslinkable segments, so that the polymerized hydrogels degrade *in-vivo.* Such hydrogels are described in U.S. Pat. No. 5,410,016, and may be useful for practicing the methods of the present disclosure.

Thus, hydrogels suitable for use in the present disclosure preferably are physically or chemically crosslinked, so that they possess some level of mechanical integrity even when fully hydrated. The mechanical integrity of the hydrogels may be characterized by the tensile modulus at breaking for the particular hydrogel. Hydrogels having a tensile strength in excess of 10 KPa are preferred, and hydrogels having a tensile strength greater than 20 KPa are more preferred. In some embodiments, biocompatible hydrogels can be used in polymerizable and non-polymerizable forms.

The hydrogel can be used as-is or further modified depending upon the desired use of the ICC scaffold. For example, the hydrogel can be derivatized with one or more different chemical groups so that the hydrogel can form bonds with other chemicals applied to the hydrogel, for example a polyelectrolyte chemical layer. In some embodiments, a polyelectrolyte can form a non-covalent or covalent bond with the hydrogel.

In some embodiments, the hydrogel can be transparent after polymerization. In some embodiments, high transparency of the ICC scaffold can be maintained even after the hydrogel is coupled with some chemical layers, biological molecules and cells. Transparency of the hydrogel permits the optical assessment of cell growth, presence of colored, fluorescent, luminescent, opalescent, phosphorescent markers and binding agents. In some embodiments, the final transparency of the ICC scaffolds can be measured using any commonly known objective measurement of transparency in plastics, containers and bottles. The method to measure transparency can be directed to measuring human perception of transparency by measuring total transmittance, transmission, haze and clarity for example using American Society for Testing and Materials (ASTM), standard ASTM D1746-03.

### Methods of making the ICC scaffold and hydrogel ICC cell scaffolds

In some embodiments, the ICC scaffolds are manufactured by first making a hexagonal array of microspheres or beads in solution as shown in FIGs. 1A -1C. Once the microspheres have settled on the well surface in their lowest energy conformation (FIGs. 1A - 1C), the microspheres can be heated sufficiently to melt, creating at the contact junctions with other microspheres. The microspheres are then cooled and templated with a solution of hydrogel. The microspheres are templated by adding a solution comprising one or more types of hydrogel monomer into and around the array to fill in the interstitial spaces and thus templating the microsphere hexagonal array to produce a 3-D hydrogel matrix. Upon polymerization and/or hardening, the microspheres are dissolved in a solvent thus leaving an inverted colloidal hydrogel scaffold containing cavities with inter-connecting pores where the microspheres are connected to one another as shown in FIGs. 1D - 1F and FIGs. 7A - 7H.

In some embodiments, the preparation of ICC scaffolds from hydrogel is carried out in five steps: (1) self-assembly of colloidal crystals from monodispersed micron-scale glass, PMMA, polystyrene or latex spheres by sedimentation; (2) annealing of the primary colloidal crystal mold to obtain rigidity of the structure and desirable diameter of the inter-connecting channels; (3) application of hydrogel into the interstitial spaces between the arrayed microspheres/infiltration and curing; (4) removal of the glass, PMMA polystyrene or latex microspheres or beads by dissolving them in solvent; and (5) thorough washing the 3-D porous hydrogel matrix with PBS buffer. Several benefits are imparted by such a preparation procedure, including the use of a hydrogel matrix, which does not require a high temperature for its production. Other advantages can include reducing the cost of manufacturing, increasing the quality, reproducibility, stability and biocompatibility of these scaffolds. These steps will be further exemplified below.

### Colloidal crystal construction

To utilize the unique geometry of ICCs as a cell scaffold the cavity size, and thus microsphere size, can be within the 50-1000 µm range. Possible strategies for constructing highly packed micro-scale colloidal crystals can include retardation of microsphere sedimentation rate and gentle agitation. These strategies can be achieved utilizing two distinct properties that micro-sized spheres possess over nano-sized spheres: effective agitation of larger volume spheres by shear force, and faster sedimentation rate of heavier spheres. In some embodiments, the microspheres can be made from any material that can form spherical bodies and which can partially melt or anneal to form junctions at the point of contact with other microspheres. In some embodiments, the microspheres comprise glass, for example, soda-lime glass, (or other glasses comprising mixtures of silicon dioxide, sodium carbonate, sodium bicarbonate, and either calcium carbonate or calcium oxide which can be dissolved without dissolution of the hydrogel matrix), latex particles, poly(styrene) and the like.

Microspheres can be introduced into a Pasteur pipette before entering into the cell-culture plate well/mold to extend the sedimentation distance. In doing so, the pipette works as a thin funnel causing a bottleneck effect for precipitating microspheres as show in FIG. 2. In some embodiments, injected microspheres can sediment one at a time. As microspheres precipitate to the bottom of the mold, gentle agitation generated by an ultrasonic bath can assist the movement of microspheres enabling the microspheres to be positioned on the substrate in their lowest energy configuration. In some embodiments, the microspheres of equal or substantially equal size can be highly packed and ordered as shown in FIGs. 1A and 1B. In some embodiments using microspheres of equal or substantially equal size, a hexagonal array can be formed according to the methods of the present disclosure as shown in FIG. 1. In some embodiments, other geometrical arrangements can be formed by allowing microspheres of different sizes to be closely packed together forming contact points with adjacent microspheres. In some embodiments, each microsphere can contact six or more microspheres positioned in three dimensions. Each layer of microspheres can serve as a template for the formation of the next layer, so when microspheres are added drop-by-drop the entire resulting structure can be seen to include microspheres having the same or substantially the same number of contacts with other microspheres as illustrated in FIGs. 1D - 1F.

In some embodiments, following preparation of highly packed colloidal crystals, the colloidal crystals can be heat-treated to partially melt the spheres. Upon slight melting, junctions are formed at points of contact between microspheres. As spheres are cooled, the junctions set, creating a solid colloidal structure. The resulting free-standing colloidal crystals are strong enough to be easily handled and removed from the well/mold. The formation of junctions prevents breakage of the crystal lattice during the infiltration of scaffolding material and ensures continuity of the chain of pores in the final scaffold. The inter-connecting pore diameter is determined at this stage because the size of melted area depends on the annealing temperature.

In some embodiments, uniformly sized soda lime glass microspheres, having diameters ranging from 50-500µm, can be used to make colloidal crystals. As a substrate, flat bottom cylindrical borosilicate glass shells can be employed, because of the higher softening temperature of borosilicate. To retard the precipitation rate, ethylene glycol can be used as a medium or solvent. In some embodiments, the diameters of poly(meth) methacrylate (PMMA) and glass beads commercially available for the preparation of ICC scaffolds can vary widely, depending on the desired application of the ICC scaffold. In some embodiments, the microspheres can range from about 50 (m to about 100 (m, from about 50 (m to about 200 (m, from about 50 (m to about 300 (m, from about 50 (m to about 400 (m, from about 50 (m to about 500 (m, from about 500 (m to about 400 (m, from about 500 (m to about 300 (m, from about 500 (m to about 200 (m, from about 500 (m to about 100 (m, and from about 500 (m to about 100 (m. In some embodiments, the colloidal crystals can be assembled by slow sedimentation of microspheres with diameters of 50, 100, 150, 200, 250, 300, 400, and 500 microns in water.

Aqueous solvent mixtures with glycerol or ethylene glycol can be used to slow down the sedimentation of microspheres and increase the geometric perfection of the scaffolds. Increasing the amount of glycerol can decrease of the speed of sedimentation and can improve the degree of order of the colloidal crystals. The same effect can also be achieved by manipulating pH and ionic strength in aqueous solutions. Increasing the electrostatic repulsion between the negatively charged beads can slow down their precipitation process and decrease van-der Waals attraction that typically results in defects. Reduction of ionic strength and elevating the pH from about 7.5 to about pH 9.0 can result in stronger electrostatic forces between the beads, thus promoting a more highly ordered array.

Infiltration of colloidal crystal molds with hydrogel. For each bead size between 50 and 500 microns, annealing of the primary colloidal crystal can be performed to impart sturdiness to the colloidal crystal mold and to create bridges between the spheres, which eventually become inter-connecting poles. For poly(meth) methacrylate (PMMA), the temperature of annealing, Tₐₙₙ, can vary between 80°C and 150°C with an interval of 100°C, and can also vary the time of annealing, tₐₙₙ. For glass beads, Tₐₙₙ can vary between 660°C and 850°C with an interval of 190°C. The higher the temperature of annealing, Tₐₙₙ, and the longer the corresponding time of the process, tₐₙₙ, the more pronounced the bridging (pore inter-connection) will be. Based on these two parameters, Tₐₙₙ and tₐₙₙ, a calibration table can be constructed that can allow a skilled practitioner to control the geometry of the scaffolds. In some embodiments, calculating the appropriate annealing temperatures and time of annealing can allow one skilled in the art to manufacture a wide array of scaffolds for individual applications and result in customizable ICC scaffolds for varying cell growth conditions.

Other methods of consolidation of microspheres under external stimulus or stimuli can be applied as well, and can include photochemical, microwave, magnetic, physical treatment and other stimuli. In some embodiments, no external stimuli may be applied.

In some embodiments, annealing can be followed by infiltration with one or more hydrogel compositions, for example, poly(acrylamide) or alginate hydrogel. In some embodiments, the hydrogel preparation can comprise one or more polymerization methods to synthesize the hydrogels. In the case of polyacrylamide hydrogels, polymerization can proceed by the addition of thermo-initiation, 10 µL of 2% K₂S₂O₈ and 0.1 mL of water being added to 0.5 mL of degassed hydrogel monomer solution, i.e. 30% w/w acrylamide monomer with some amount of a cross-linking agent, for example multifunctional crosslinkers such as ethylene glycol dimethacrylate (EGDMA), *N,N'* methylenebisacrylamide (NMBA), 1,4 butanediol dimethacrylate (BDMA) and trimethylolpropane triacrylate (TMPTA) as cross-linking agent. The mixture can be infiltrated into the primary colloidal array and then polymerized for varying temperatures and times, depending on the percentage monomer and concentration of cross-linking agent. In some embodiments, the acrylamide hydrogel can be polymerized at 70°C for 12 hours. For redox initiation, 0.5 mL of monomer solution, 0.1 mL of 0.05M L-ascorbic acid and 10 µL of 2% K₂S₂O₈ can be mixed. The hydrogel mixture can be infiltrated into the primary colloidal crystal array, and polymerization can be carried out to completion at room temperature for 12 hours. The resulting gel can then be soaked in tetrahydrofuran (THF) to remove the polymeric colloid array comprising the microspheres. The inverted hydrogel scaffold can then soak in water and can reach an equilibrium swelling state at room temperature.

In some embodiments, the glass beads can be removed by soaking in 0.5% hydrofluoric acid (HF) with subsequent thorough rinsing to dissolve the glass beads and leave the polymerized hydrogel matrix intact. Wash steps can be employed to remove the HF until the concentration of F- falls below the concentration of fluoride in de-ionized water (approximately <10⁻⁵M). After the cavities have been formed by dissolving the glass microsphere in the hydrogel, the cavities can be expected to have between 3 to about 12 pores per spherical cavity. The hydrogel matrix can comprise from about 50% to about 90% porosity by volume of the matrix.

The geometrical characteristics of the hydrogel scaffolds can be evaluated and verified using confocal microscopy in addition to environmental scanning electron microscopy (SEM), which does not cause drying of the hydrogel. The diameters of spherical cavities formed in place of the microspheres and the diameters of inter-connecting pores formed in place of interparticle contact junctions can be measured and compared to the parameters of the original colloidal particles. The empirical dependence between Tₐₙₙ and tₐₙₙ, and the diameter of inter-connecting pores can be determined and selected, ranging in size between 50 and 500 nm.

### Automatic colloidal crystal construction system

In another embodiment, the present disclosure is directed to an apparatus for the use in the production of ICC scaffolds and hydrogel ICC cell scaffolds comprising a 3-D porous ICC scaffold having cavities, wherein the cavities each have inter-connecting pores as described herein.

The apparatus of the present disclosure can be described with reference to FIG. 3. In FIG. 3, an apparatus for producing ICC scaffolds having a porous hydrogel 3-D matrix is illustrated. The apparatus comprises a commercially available glass vial well plate 10 operably mounted on the surface of a table 20. The glass vial well plate 10 consists of a metal base 30 with spaces to fix cell culture flat bottom glass vials 40 in 12 rows of 8 vials. The glass vials 40, with inner diameters ranging from 5-7 mm, possess the same dimensions as wells in a standard cell culture well microplate, and serve as molds for colloidal crystals. The glass vial well plate 10 sits in an ultrasonic bath 60 mounted on the table 20, so that the bottom ends of the glass vials 40 are submerged in the bath.

A plurality of dispensers, for example, without limitation Pasteur pipettes 80 can be secured to each glass vial 40 to ensure slow sedimentation of microspheres into the glass vial 40. The Pasteur pipette 80 can be centered in the opening of each glass vial 40, and placed so that its tip is within the glass vial 40. The pipette 80 and glass vial 40 can be filled with ethylene glycol obtained from one of a plurality of reservoirs 100 to allow for slow sedimentation of microspheres through the pipette 80 and into the glass vial 40.

A uniform quantity of glass microspheres is preferably injected into each glass vial 40. To obtain uniform microsphere distribution, an automated microplate pipetting system 200 is used to deliver accurate volumes of microspheres, reagents, hydrogel solution and wash solutions. An automated microplate pipetting system 200 consists of 8 micropipette tips aligned in a row 220, spaced identically as the 8 wells in each row of a cell culture well plate, for example in microplate 10. The automated microplate pipetting system 200 is positioned above the Pasteur pipettes 80 and vials 40 so that a consistent quantity of glass microsphere dispersion from a microsphere reservoir 240 is dropped simultaneously in each of the eight Pasteur pipettes 80 in a row. After simultaneously releasing a drop of microspheres into each of the 8 pipettes 80, the automated system moves to the next row. This is repeated for each of the 12 rows, and then the automated system is timed to rest for 15 minutes before dispersing another drop in each pipette. The apparatus can also comprise a timing means such as an electronic, digital or analog timing mechanism to actuate the some components, including the automated microplate pipetting system 200, the ultrasonic bath 60, and the oven 260 and alarm systems (not shown). A 15-minute gap between each drop release can be designed to ensure microspheres sediment slowly and find their lowest energy configuration, forming a hexagonal close-packed array 300, before the next drop is added. Once the colloidal crystal array has reached the desired height (from about 0.3 to about 1.5mm), Pasteur pipettes 80 are removed, and microplates 20 are left under gentle agitation in the ultrasonic water bath 60 for 4-5 hours without further addition of microspheres as shown in FIGs. 4A and 4B.

### Automatic drying and annealing system

The glass vial well plate containing cell culture molds is transferred either manually or robotically to an oven 260 preset to a temperature ranging from about 120°C to about 170°C for about 10-15 hours to evaporate all solvent, leaving dry, un-annealed colloidal crystals. The temperature can be gradually increased to a range from about 660°C to about 850°C, depending on the size of microspheres, for about 2-3 hours to anneal the microspheres together, forming a solid colloidal crystal array. The solid colloidal crystal array can serve as a template for the ICC. The oven temperature can be set and changed by a timer.

### Automatic hydrogel infiltration and polymerization system

The glass vial well plate 10 can be removed from the oven manually or robotically and placed on the apparatus table 20 or ultrasonic bath 60 for further liquid manipulation steps described herein. The automated microplate pipetting system 200 injects a hydrogel precursor solution into the vials 40 containing colloidal crystals, under slight agitation in the ultrasonic bath 60 to ensure complete infiltration. Once the hydrogel precursor solution has filled the entire volume of the colloidal crystal, the colloidal crystals can be removed from the molds and put between two highly absorbable sponge sheets. By briefly pressing down on the colloidal crystals from opposite directions, precursor solution at the top and bottom of colloidal crystals can be effectively removed; precursor solution remains in the inner space or interstitial spaces of colloidal crystals by capillary force. Next, colloidal crystals are exposed to UV light 340 for 12 hours to polymerize the hydrogel precursor solution.

### Automatic microsphere dissolving and washing system

The colloidal crystals infiltrated with polymerized hydrogel can be transferred to a plastic bath 350 on the apparatus table 20 containing a solution derived from reservoir 360 containing for example, 1% HF, using an automated liquid dispensing means operably connected to a power source and pump to retrieve solution from one or more of the plurality of reservoirs 100. The colloidal crystals can be stirred periodically or continuously for approximately 2 days using an automatic stirrer such as a magnetic stirrer. The automated pipetting system 200, can periodically remove solution from the plastic bath 350 and replace the retrieved solution with fresh HF solution obtained from reservoir 360 in an equal or different volume. The washing system is designed to continuously remove and replenish 1% HF. After microspheres are dissolved from the hydrogel, an inverted replica of the colloidal crystal remains, which is a hydrogel ICC. Hydrogel ICCs can be removed from the 1% HF solution, and placed into a circulating bath 350 of deionized water for 24 hours, which is obtained using the automated pipetting system 200, from reservoir 380. Water is removed and then the hydrogel ICCs can be washed in a solution of phosphate buffered saline contained in reservoir 400 to neutralize any remaining HF using the automated pipetting system 200. The ICCs can then be rinsed again in deionized water obtained from reservoir. In some embodiments the ICC scaffolds can be made by cutting out large sheets or cylinders of ICC scaffold matrix made by self-organization of colloidal spheres followed by their infiltration with biocompatible polymer precursor for example a hydrogel polymer precursor, followed by removal of the beads. Cutting from a large piece of the hydrogel matrix will significantly accelerate the production process and will allow one to reduce the time and cost to prepare the scaffolds.

### ICC scaffold surface coating through the layer-by-layer (LBL) method

In some embodiments, methods are described to functionalize the surface of the ICC scaffold using a layer-by-layer (LBL) approach. The LBL method can be adaptable to any chemical process and allows functionalization of the scaffolds with any kind of biocompatible material individually, sequentially or as a mixture following virtually the identical procedure while retaining their biological activity.

In some embodiments, the LBL method is also known as polyelectrolyte multilayers (PEM) and electrostatic self-assembly. In some embodiments, the LBL method comprises sequential dipping of a substrate having contained therein an ICC scaffold into a solution of oppositely charged species alternating with water rinse. The first rinse can be any charged polyelectrolyte species. The polyelectrolytes can be any ionic solution capable of forming a layer on external and/or internal surfaces of the hydrogel scaffold and/or a previously coated polyelectrolyte layer, depending on the deposition or layering method. In non-limiting examples, the polyelectrolyte can be clay followed by poly(dimethyldiallylammonium) chloride (PDDA). Clay possesses a negative charge, and can therefore serves as a negative polyelectrolyte, while PDDA possesses a positive charge, and is a positive polyelectrolyte. In some embodiments, the polyelectrolyte can be any charged mixture or pure species, including without limitation, (PDDA), alumosilicate clay (montmorillonite), ionic polymers, for example, poly-lysine, oligonucleotides, poly acetylamine, collagen, alginate, carageenan, fibronectin, gelatin, extra-cellular matrix, poly(ethyleneimine) (PEI), poly(allylamine hydrochloride (PAH), poly aniline, polyacrylic acid, poly lactic acid, compositions containing cellulose, for example, cellulose nanocrystals, and carbon nanotubes.

In some embodiments, the ICC scaffold can be contacted with the polyelectrolyte in any manner commonly used in porous structure coating methodologies. For example, the ICC scaffold can be sprayed, dipped, washed or coated with the one or more polyelectrolytes or electrostatically attracted inside the scaffolds, using for instance electrocapillari phenomena or electrostatic attraction of the LBL component to external electrode. ICC scaffold itself may be made conductive by a producing a conductive coating on it, and thereby replace any additional electrode. For example, the ICC scaffold can be sprayed, dipped, washed with the one or more polyelectrolytes. In each dipping cycle, a (mono)layer of the species to be applied, adsorbs to the scaffold, while the rinse step removes their excess. The next dipping cycle results in enhanced adsorption of the oppositely charged species, which is also accompanied by a switch in the surface charge. This promotes the adsorption of the subsequent layer. Due to the monomolecular nature of the layers deposited in each cycle, the LBL technique affords nm scale precision in thin film thickness. This cycle can be repeated as many times as one need to build up a multilayer to a desirable thickness. The process can be easily automated and scaled-up. Importantly, the assembled biopolymers retain their 3-D structure and biological activity.

In some embodiments, the ICC scaffolds can be coated with a variety of proteins from extracellular matrix (ECM), including without limitation, biopolymers such as collagen and fibronectin. It is contemplated, that the deposition of polyelectrolyte can improve the overall density of the cells seeded into and around the ICC scaffold.

The internal and/or external surfaces of ICC scaffolds can be coated with biologically functional molecules via LBL assembly. In some embodiments the ICC scaffolds can be coated in situations where there are large numbers of ICC scaffolds to be coated. In some embodiments, a different method can be applied to coat ICC scaffolds individually. In some embodiments, a coating method can be used to produce ICC scaffolds having surfaces that are biologically active and promote cell attachment, but are not specific to a cell type or function, such as directed differentiation or increased proliferation. In other embodiments, a second coating method can be used to produce ICC scaffolds coated with biomolecules intended for promoting attachment, growth, proliferation, or differentiation of a specific cell type. This second method is noted because as the intended function of the ICC scaffold becomes more specific, it may be more economical to utilize a method intended to produce smaller numbers of scaffolds.

### LBL coating for bulk ICC scaffolds

In some embodiments, methods for coating large numbers of ICC scaffolds having surfaces that are biologically active and promote cell attachment comprise the step of placing all of the scaffolds into one or more polyelectrolyte solution sequentially. The general aim of LBL on the surface of an ICC scaffold is to promote cell attachment. In some embodiments, the LBL bulk-coated ICC scaffolds, are coated with the components chosen on the grounds of improved functionality and economic practicability.

Methods for LBL coating of bulk ICC scaffolds are described herein. First, ICC scaffolds can be placed in a bath of water to remove excess monomer. The bath can contain a built-in magnetic stirrer with adjustable stirring speeds, as well as a drain and water source to continuously replenish fresh water. In each step, the stirring bath serves to assist in diffusion of water or polyelectrolyte solution. The scaffolds can be placed into a rectangular metal net having the same dimensions as the bath. The scaffolds and net can be dipped into the bath vigorously stirring the scaffolds in the bath for approximately 30 minutes. Next, the scaffolds can be collected by removing the net from the bath and letting water drip from the net. The net containing the scaffolds can be transferred to a similar stirred bath of 0.5% PDDA solution for approximately 30 minutes. The scaffolds are then collected and transferred back to the water bath for about 15 minutes of rinsing, to rinse excess PDDA and ensure a monomolecular layer remains. The scaffolds are then collected and transferred to a 0.5% clay bath and stirred for another 30 minutes. After coating with PDDA, rinsing with water, coating with clay, and rinsing with water, the scaffolds are considered to have received one layer. In an exemplary embodiment, the coating and washing steps can be repeated at least five times so that five layers can be coated on the scaffold surface. After the fifth layer is applied, the scaffolds are replaced in a water bath for storage. Since the number of layers to be coated on the ICC scaffold can vary, the number of steps can vary accordingly. Similarly, the duration of the coating and washing steps can easily be adjusted according to the coating's composition and its intrinsic capability to adhere to the layer applied before it. In lieu of clay, one can also use other colloidal materials, for example, nanocolloids of cellulose in different varieties, dispersions of extracellular matrix, proteins, carbon nanotubes, nanoparticles, and other adhesion promoting materials. Also, the scaffolds can be coated with nanometer scale layer(s) of biocompatible materials facilitation specific cellular response by reaction in the bulk of the fluid infiltrating the scaffold. These types of coatings can be applied directly onto the polymer, for example a hydrogel, or on LBL layers serving as a substrate for subsequent coating of the scaffolds. In some embodiments, coatings comprising SiO₂ by controlled hydrolysis of its precursors or by calcium phosphate by precipitation reaction of two salts. Both coatings are expected to enhance cellular adhesion. One of ordinary skill can experiment and obtain optimal incubation times for the particular coating required without undue experimentation.

### LBL coating for individual ICC scaffolds

In embodiments requiring the application of layers of polyelectrolytes to small numbers scaffolds or individual ICC scaffolds, polyelectrolyte coating is not done in large containers. In some embodiments, ICC scaffolds to be coated in this smaller scale process, can be treated with clay/PDDA as described above, to impart the benefit of increased cell attachment as well as greater biomolecular activity. To treat and coat fewer scaffolds, in accordance with the present embodiment, an ultrasonic bath can be used to assist diffusion, rather than a stirring bath. In some embodiments, the polyelectrolyte and/or washing solutions can be dispensed manually using for example a pipette or other similar apparatus, or the solutions can be dispensed automatically. In some embodiments, the automated microplate pipetting system can be used to dispense the polyelectrolyte and/or washing solutions. First, ICC scaffolds can be placed into one or more wells of cell culture well plates (having a number of wells ranging from 1-1536). The automated microplate pipetting system can introduce the first polyelectrolyte solution ranging from several mL to several microliters into the well or wells of the cell culture plate. Gentle sonication can be applied for 15 minutes to facilitate diffusion of the coating materials into the 3-D ICC scaffolds, while at the same time prevent damage to the formerly coated film. Next, the polyelectrolyte solution can be removed from the well, and deionized water can be added into the well and gently sonicated for about 30 minutes to remove excess polyelectrolyte.

After removing the wash water, a solution of polyelectrolyte or a bioactive agent, can be added to the ICC scaffolds. Note that the polyelectrolyte can be chosen to possess an opposite charge to that of the desired bioactive agent. Lastly, the bioactive agent solution can be removed and water can be added to the scaffolds to rinse the excess bioactive agent. This process generally requires only 1-5 applications of polyelectrolyte/bioactive agent to achieve surface activity.

In some embodiments, one or more bioactive agents can be added to the hydrogen ICC scaffold to render the scaffold biocompatible and/or tissue selective, i.e. possessing the required biological molecules which can influence an attached cell to grow, perform a cell function such as differentiation or activate or repress an internal or external cell signal. In some embodiments, the ICC scaffold can further contain one or more added bioactive agent, either: (1) encapsulated in one or more hollow space(s) within a "hollow" void; or (2) located within or throughout the bulk of a "solid" particle, or of a core, wall, or layer of a hollow or laminar particle, or surface or wall of a void and/or pore. Examples of bioactive agents for use in an embodiment of the present disclosure can include: bone morphogenic proteins (e.g., BMP1-BMP15), bone-derived growth factors (e.g., BDGF-1, BDGF-2), transforming growth factors (e.g., TGF-α, TGF-β), somatomedins (e.g., IGF-1, IGF-2), platelet-derived growth factors (e.g., PDGF-A, PDGF-B), fibroblast growth factors (e.g., αFGF, βFGF), osteoblast stimulating factors (e.g., OSF-1, OSF-2), and sonic hedgehog protein (SHH); notch protein, other hormones, growth factors, and differentiation factors (e.g., somatotropin, epidermal growth factor, vascular-endothelial growth factor; osteopontin, bone sialoprotein, α 2HS-glycoprotein; parathyroidhormone-related protein, cementum-derived growth factor); biogenic proteins and tissue preparations (e.g., collagen, carbohydrates, cartilage); gene therapy agents, including naked or carrier-associated nucleic acids (e.g., single- or multi-gene constructs either alone or attached to further moieties, such as constructs contained within a plasmid, viral, or other vector), examples of which include nucleic acids encoding bone-growth-promoting polypeptides or their precursors, e.g., sonic hedgehog protein (see, e.g., P C Edwards et al., Gene Ther. 12:75-86 (2005)), BMPs (see, e.g., C A Dunn et al., Molec. Ther. 11(2):294-99 (2005)), peptide hormones, or anti-sense nucleic acids and nucleic acid analogs, e.g., for inhibiting expression of bone-degradation-promoting factors; pharmaceuticals, e.g., medicaments, anti-microbial agents, antibiotics, antiviral agents, microbiostatic or virustatic agents, anti-tumor agents, and immunomodulators; and metabolism-enhancing factors, e.g., amino acids, non-hormone peptides, toxins, ligands, vitamins, minerals, and natural extracts (e.g., botanical extracts). The bioactive agent preparation can itself contain a minority of, e.g., processing, preserving, or hydration enhancing agents. Such bioactive agents or bioactive agent preparations can be contacted or applied into and onto the ICC scaffold through any dispensing means, for example, diffused, sprayed, suctioned, imbibed or added to the polymer solution directly before forming the three dimensional matrix, or combinations thereof. Where both the void and pore surface and polymer solution contain bioactive agent(s), the bioactive agent(s) can be the same or different. It should be appreciated however, that the present disclosure is not limited by any particular method of treating the ICC scaffold with a bioactive molecule, for example a growth factor, and the disclosure is applicable to any such method now known or subsequently discovered or developed.

In some embodiments, growth and/or differentiation factors useful in the present disclosure can include, but are not limited to: sonic hedgehog, notch ligand, vascular endothelial growth factor (VEGF), epidermal growth factor (EGF), fibroblast growth factor (FGF), insulin growth factor (IGF), erythropoietin (EPO), hematopoietic cell growth factor (HCGF), platelet-derived growth factor (PDGF), nerve growth factor (NGF), transforming growth factors (TGF-α and TGF-β), bone morphogenetic protein 1-17 (BMP 1-17) or combinations thereof.

### Incorporation of the Biologically Active Agents in the 3-D ICC Scaffold.

Biologically active agents can be applied to the hydrogel matrix of the scaffolds before or after placement in the well-plates. Scaffolds can be contacted with a desired chemical or biological active material, to be incorporated in the wall, cavities and pores of the 3-D hydrogel matrix using for example, vacuum suction, spraying, immersing in a bath or wetting techniques. After a desired incubation period, the chemical or biological active material can be removed and scaffolds will retain a specific amount of the chemical or biologically active material due to entrapment/adsorption in/on the hydrogel matrix. In some embodiments, the bioactive agent can be added to one or more polyelectrolytes used to coat the scaffold by the LBL procedure.

### Transfer to cell culture well microplate and packaging

In some embodiments, the present disclosure includes two methods of packaging the ICC scaffolds. In some embodiments, a method for storing hydrated ICC scaffolds can be employed. The final ICC scaffold samples can be placed in one or more cell culture well microplates with a compatible sterile solution, for example, deionized water or phosphate buffered saline solution. The cell culture well-plate can then be covered by a sealing tape as shown in FIG. 5. After the cell-culture well plate is sealed the scaffolds can be sterilized using any compatible and convenient means, for example, sterilization under UV or radiation, gamma-radiation, electron beam or the like for up to 12 hours, so that the scaffolds are ready-to-use. In some embodiments, the sealed scaffolds can be sterilized chemically, for example with ethylene oxide and chloride dioxide. The ready-to-use sterilized hydrogel scaffolds can be stored in a 4°C refrigerator prior to delivery and use.

The second method is to pack ICC scaffolds after a dehydration process, for instance freeze-drying also known as lyophilization. In some embodiments, ICC scaffold samples are immersed in liquid nitrogen for 5 min, and then placed and lyophilized in a freeze drying machine for about 12 to about 24 hours. This process minimizes the shrinkage of ICC scaffolds, curtailing damage of coated materials. Dehydrated ICC scaffolds can be temporarily glued in a cell culture well-plate utilizing 50:50 poly(lactic-co-glycolic acid) (PLGA) polymer. In some embodiments, the cell culture plate can also be covered by a sealing tape. The ICC scaffolds can be sterilized using a chemical gas or sterilized under UV radiation for approximately12 hours and stored in a room temperature desiccator. Dehydrated scaffolds can easily intake deionized water or phosphate buffered saline solution within one hour, and thereby recovering its original biological and physical properties. Because the scaffolds can be glued with PLGA, the scaffolds can be stationary during the re-hydration process. The second desiccation method can be tailored for long-term storage of ICC scaffold samples.

### Methods of use

ICC scaffolds are ideally suited as a 3-D cell culture substrate because of its highly porous and mechanically stable structure. The highly ordered and uniformly sized porous geometry can be replicated with great consistency, and can be made adjustable by altering the microsphere size and annealing temperature which can control the size of the cavities and inter-connecting pores. In some embodiments, the internal and external surfaces of an ICC scaffold can be coated with one or more than one biological molecules utilizing a layer-by-layer (LBL) molecular assembly technique that can be used for coating oppositely charged polyelectrolytes. A large variety of biomolecules can be stably deposited and applied to the surfaces of the pores and of the surfaces of the three dimensional matrix through the LBL method with minimal loss of bioactivity. Low mass transport resistance within the ICC structure permits a uniform coating and ultra-thin multilayers on the complex 3-D porous substrate with nanometer precision. As a result, LBL-coated ICC scaffolds have precisely designed micro- and nano-scale geometry and surface properties. Due to its simple and robust fabrication procedure, a consistent 3-D microenvironment can be maintained.

### Preparation of ICC cell scaffolds

In some embodiments, the ICC scaffolds described herein can be used to selectively grow and culture living cells. As used herein, living cells can include bacteria, algae, yeast, plant cells and animal cells. In some embodiments, the living cells can be selected from the group consisting of myocyte precursor cells, cardiac myocytes, skeletal myocytes, satellite cells, fibroblasts, cardiac fibroblasts, hepatocytes, chondrocytes, osteoblasts, removal cells, endothelial cells, epithelial cells, embryonic stem cells, hematopoietic stem cells, neuronal stem cells, hair follicle stem cells, mesenchymal stem cells, and combinations thereof. Preferably, the living cells are mammalian cells including for example, rabbit, dog, goat, horse, mouse, rat, guinea pig, monkey, and human cells. Still preferably, the mammalian cells are human cells. In some embodiments, ICC scaffolds can be prepared as described above and can be tailored for the growth or many different types of living cells, the growth of specific types of cells, or enable one or more cell types to become differentiated into a different lineage of cells.

In some embodiments, the ICC scaffolds can be seeded with living cells using any commonly known cell seeding technique, including without limitation, a liquid dispensing means to aseptically transfer cells from one container to the well containing the ICC scaffold, for example, a pipette, spraying a cell culture onto and into a ICC scaffold, filtering a cell culture through a ICC scaffold and by centrifuging a cell culture solution on top of a ICC scaffold and combinations thereof. In some embodiments, the population of stem cells and other progenitor cells, for example hematopoietic stem cells and mesenchymal stem cells, can be recruited to the ICC scaffolds with the use of stem cell specific chemokines and chemoattractants. For example, when the ICC scaffolds of the present disclosure are implanted into a subject *in-vivo,* a desired population or populations of stem cells or progenitor cells can be induced to populate the scaffold by coating on a surface of an ICC scaffold void and/or pores with one or more stem cell or progenitor cell specific chemokine or chemoattractant. In some embodiments, the ICC scaffold can be coated with Stem Cell Factor-1 (SCF-1) at concentrations ranging from 1 ng/mL to about 100 µg/mL using the LBL method described above. In other embodiments, the chemokine or chemoattractant can be applied to the coated ICC scaffold using any approach described herein, including, for example, spraying, dipping, vacuum induction and the like to obtain an ICC scaffold having at least a surface of a void and/or a pore coated with one or more appropriate chemokines and/or chemoattractants. In some embodiments, other cell types can be induced to migrate from the host tissue or circulation (blood or lymphatic) into the ICC scaffold, for example, B-cells, T-cells, macrophages, monocytes, NK cells, and other lymphocytes, dendritic cells, neutrophils, basophils, eosinophills, platelets and mast cells. In some embodiments, as an illustrative example, neutrophils (PMNs) can be induced to migrate into the ICC scaffolds using interleukin 8 (IL-8). One of ordinary skill in the art can formulate specific chemokines and/or other chemoattractants, for example, ligands of some chemokine receptors that may be found on the surface of the desired cell type (e.g. CXCR1-CXCR7 and CCR1-CCR11) which are known to function as chemoattractant molecules for a specific cell-type or cell-types. Concentrations of each of these chemoattractants can be empirically determined without undue experimentation.

In some embodiments, the cell culture plates can contain identical ICC scaffolds having identical matrix coatings but different cell culture conditions, for example, different culture media. Alternatively, the cell culture plates can contain identical ICC scaffolds having different matrix coatings but identical cell culture conditions, for example, each well having a different biological molecule adhered to the polymer matrix during the LBL process. In these embodiments, each well can contain the same media. The result of analysis of cell behavior in each well will allow the experimentalist to choose optimal conditions for specific biological system or a specific cell type.

In some embodiments, cells can be cultured in ICC scaffolds further comprising a recirculating media system. The cell culture plates may also have specially engineered channels and/or supply mechanisms that can facilitate the delivery of nutrients to all the parts of the scaffold and especially to the bottom of the scaffold. An ICC having a recirculating media system is shown in FIG. 6.

### Maintenance and Expansion of Stem cells

In some embodiments, implementation of cell culture microplates with ICC scaffolds for selecting stem cell conditions for proliferation and differentiation can be made. In some embodiments, the ICC scaffold can be layered or coated with one or more desired biological molecules, including growth factors and receptor ligands, and seeded with one or more stem cells, including embryonic stem cells, hematopoietic stem cells, neuronal stem cells, mesenchymal stem cells, and hair follicle stem cells. The stem cells useful in the present disclosure can be derived from autogeneic, allogeneic or xenogeneic sources. In some embodiments, the cell culture media in wells will be varied and the reaction of stem cells on the presence or absence of specific components in the ICC scaffold or tissue culture media can be analyzed. According to these results, the choice for specific biological molecules, including growth factors and different media components for optimal stem development and/or expansion can be made.

In some embodiments, *in-vitro* expansion of stem cells, for example, without limitation, embryonic stem cells, hematopoietic stem cells, neuronal stem cells, mesenchymal stem cells, and hair follicle stem cells generally involve techniques that utilize stromal cell support, growth and differentiation factors and/or addition of cytokines. In another embodiment of the present disclosure, expansion of the hematopoietic stem cell ("HSC") population without induction of maturation or differentiation of the cells can be accomplished by culturing the HSCs in the presence of bone marrow stromal cells, for example, HS-5 cell line or any primary cell line isolated from a stem cell tissue source, for example, bone marrow or adipose tissue, can be seeded on the ICC scaffolds within a rotary cell culture system bioreactor. In some embodiments, one or more primary cell lines that can be isolated from bone marrow or other sources of stem cells (e.g. adipose tissue, chord blood or hair follicles) which can support the one or more stem cell lineages to reproduce and/or differentiate into any desired cell lineage with the appropriate biological signals known in the art of stem cell differentiation, can be used. In some embodiments, precursor stem cells that are differentiated into bone forming or bone remodeling cells, for example, osteoblasts, osteoclasts and chondrocyte cell lines can be used in a supporting role for stem cells production and differentiation. In some embodiments, primary cell lines that represent some support cell types such as stromal cells, osteoblasts, adipocytes and the like can be used to support the growth and/or differentiation of a specific stem cell type.

In some embodiments of the present disclosure, 3-D ICC scaffolds can be particularly useful for the structural support for the cellular assays which may be used for the development of different vaccines and antibody or cell based therapeutic compositions and biologics. CD34+ stem cells can serve as precursors to a number of hematopoietic cells including B-cells developing in the bone marrow. Differentiation of HSCs into pro-B cells and finally into pre-B cells is a stepwise progression that requires sequential expression of lymphoid regulatory genes as well as somatic rearrangements of the immunoglobulin heavy and then light chain genes. Rearrangements of the light chain genes are followed in immature B cells by the expression of cell surface IgM. Mature B cells express both IgM and IgD on their surface and it is at this stage that the mature but antigen-naive B cell exits the bone marrow and enters the peripheral circulation.

In some embodiments, a cellular culture with a subset of immune cells and/or HSCs can be cultured in the wells containing an ICC scaffold. The addition of pro-B-cell lineage growth and differentiation factors can elicit a phenotypic differentiation into one or more B-cell subsets. In some embodiments, exposure of one or more specific antigens administered either in the culture media, or tethered to the ICC scaffold seeded with a mixed B-cell population can be used to gather knowledge of the antigen-immune response. A greater understanding between the cells of the immune system and a particular antigen can permit rational design of antigen structures for vaccine development. The analysis of this reaction will enable optimization of the vaccine composition and methods of its preparation.

### Drug and Pharmaceutical testing and evaluation

In addition to some antigen preparations, pre-existing and candidate compounds can be tested for biological activity or toxicity using *in-vitro* and *in-vivo* constructs employing ICC scaffolds seeded with cells. Designed drug candidates with individual chemical structures, as well as some drug formulations, such as hepatoxic or hepatoprotective drugs, vaccines, anticancer drugs, antiviral drugs and others, can be tested initially on cell cultures in order to maximize potential curing effects and evaluate the potential toxicity, prior to animal and human trials. The overall research and development cycle for drugs costs $300-800 million in capital and up to 10-12 years in time. One of the reasons for such great cost is that the vast majority of drug candidates are screened out at the stages of animal and human trials. More efficient methods of testing of drugs at any stage of drug development, particularly at the stage of *ex-vivo* studies, which are substantially less expensive than animal and human testing cycles, will lead to acceleration of drug discovery, reduction of the cost of pharmaceutical development, and better drugs. This particularly true for advanced drugs for HIV, cancers, metabolic, immunological and autoimmune deceases. Efficacy of *in-vitro* testing can be significantly improved provided that better *ex-vivo* models for different organs and tissues are developed. A large body of research indicates that cultured cells organized in three-dimensions (3-D) behave a lot more closely to the original tissues and retain more natural functions than the cells in 2D cultures. Driven by the desire of the pharmaceutical industry to replace some portion of regular *in-vitro* drug testing and potentially some animal trials with experiments in 3-D cell cultures, which can reduce the cost and the time from inception to production of a new drug, much work is also being done on the development of replicas of key body tissues.

A large variety of materials and manufacturing processes have been used to make 3-D scaffolds for previous 3-D *in-vitro* drug testing studies. However, many of them are not convenient for mass drug testing protocols due to strong light scattering/absorption and variability in the scaffold quality/topology. Virtually all of the commercially made 3-D scaffolds are made from ceramics or other inorganic materials and are difficult to incorporate in the developed drug evaluation protocols. Several advantages can be afforded to transparent ICC scaffolds of the present disclosure, and can include ease of monitoring and examining changes in cell function after administration of a compound for example, a toxin, a pharmaceutical, a drug, or a candidate compound to the cells in the ICC scaffold.

In some embodiments, the present disclosure further contemplates a method of identifying the effects of a test compound which can include one or more of a toxin, a drug, a biologic, a medicament, a pharmaceutical composition, or an infectious agent (for example a protein, for example, a prion or other self-replicating protein or peptide, a bacterium or viral pathogen) on cell function comprising administering a compound *in-vitro* to an inverted colloidal crystal scaffold seeded with viable cells; and determining the affects of the compound on the living cells by measuring, collecting, or recording information on the cells or products produced by the cells. In some embodiments, the cells to be tested can be any mammalian cell including without limitation, myocyte precursor cells, cardiac myocytes, skeletal myocytes, satellite cells, fibroblasts, T-cells, B-cells, dendritic cells, macrophages, monocytes, neutrophils, mast cells, basophils, eosinophils erythrocytes, cardiac fibroblasts, hepatocytes, chondrocytes, osteoblasts, endothelial cells, epithelial cells, embryonic stem cells, hematopoietic stem cells, neural cells, neuronal cells, hair follicle stem cells, mesenchymal stem cells, and combinations thereof. In some embodiments, the cells to be studied include bone marrow cells, cardiac myocytes, hepatocytes and neural cells.

In some embodiments, the method further comprises determining the effects of a test compound on the cells by measuring or identifying changes in cell function. This can be accomplished by many methodologies known to those skilled in the art including, for example, Western blot analysis, Northern blot analysis, RT-PCR, immunocytochemical analysis, flow cytometry, immunofluorescence, BrdU labeling, TUNEL assay, and assays of enzymatic activity, and automated assays encompassing the above measurement assays in a highly sequential manner high throughput and high content analysis, for example nucleic or protein chip arrays using fluorescently labeled molecules.

In some embodiments, the living cells are hepatocytes. Accordingly, measurements of parameters such as albumin production and liver enzyme activity can be made. By way of example, the instant ICC scaffold containing hepatocytes clustered into spheroids that can be cultured indefinitely in the ICC scaffold could be treated with one or more test compounds, such as a liver toxin, a drug and/or pharmaceutical, for example, statins, or natural or synthetic compounds, for example cholesterol or lipoprotein. The effects of the test compounds can be determined by measuring the change in the production of albumin, cholesterol, detoxifying enzymes, and other liver enzymes, which can be measured as described above. This method provides an *in-vitro* diagnostic system that can be utilized to rapidly assay the physiological consequences of administration of a given drug, pharmaceutical composition, medicament or toxin on cell function such as, production of albumin and liver enzymes.

In some embodiments, testing of the drugs, pharmaceuticals, biologics, toxins, proteins/peptides affecting the brain and Central Nervous System (CNS) can be modeled *in-vitro* using ICC scaffolds cultured with the appropriate target cells or tissue. In some embodiments, the target tissue or cells are neural tissue or neural cells. Currently experimental 3-D models for neural tissues are not available. 2-D neural cell cultures lack exceptionally important connectivity components along the cells, which are targeted by many drugs. Also the ICC scaffolds of the present disclosure are contemplated to be exceptionally helpful in the understanding of cellular interactions between different cells in neural tissue, such as the cellular interactions between neurons, oligodendrocytes, glial cells, astrocytes, Schwann cells and the like particularly useful to study the pathological processes in neurological diseases, for example as in Alzheimer disease and Parkinson's disease.

In some embodiments, a diagnostic assay or system can be realized comprising administering a pathogen or infectious agent, for example, a prion or other self-replicating peptide, an amyloid-beta peptide, a bacterium or virus, to human or animal cultured cell or cultured cells, for example neuronal cells, hepatic cells infected with a hepatitis virus or T-cells infected with human immunodeficiency virus (HIV).

### Production of Artificial Tissue.

In some embodiments, the ICC scaffolds can be manipulated to provide scaffolds enhanced for cellular infiltration, integration and remodeling of introduced cells. In some embodiments, the ICC scaffolds of the present disclosure can be utilized to grow any living cell as enumerated and described above. The ICC scaffolds of the present disclosure can be made to grow and reconstruct living tissue material. In some embodiments, the tissue can be artificial skin, hair follicles, blood vessels, bone marrow, neural tissue, muscle, cardiac muscle, liver tissue, bone and cartilage. Cells used for reconstruction of autogenous or allogeneic tissue can be of any type typically residing in the tissue type to construct. In some embodiments, stem cells can be used to provide the progenitor source of cells to be grown *in-vitro.* Stem cells are ideally suited for the construction of autogenous and allogeneic tissue because they can be readily isolated from the patient, for example, mesenchymal stem cells from bone marrow, skin stem cells from the dermis, adipose derived stem cells from lipectomy procedures (liposuction), hair follicle stem cells from hair transplants. In some embodiments, embryonic stem cells from mammalian sources, including for example human embryonic stem cells can be used to create any tissue type in an artificial construct using ICC scaffolds. For the purpose of regeneration of tissues ICC scaffolds can be made from biodegradable materials such as PLA, PLGA, hyaluronic, acid, collagen, etc.

Some stem cell growth and differentiation factors are relatively well known and have been successfully used to produce differentiated cells from progenitor and stem cells *in-vivo* and *in-vitro.* It is contemplated in the present disclosure, that artificial tissue can be used to graft and repair defective tissue due to disease and trauma, to replace tissue to correct a congenital aberration and to enhance and augment cosmetic procedures.

### Artificial Ex-Vivo Bone Marrow Constructs

The open geometry of the ICC lattice, high porosity (having 50-90% free space), and large surface area make ICC an attractive structure for growth and differentiation of bone marrow cells which can include progenitor and mature cells found in the bone marrow of mammalian species, preferably human bone marrow. Furthermore, the ICC lattice provides ideal microenvironments for studies of 3-D effects in cell cultures. It is also apparent that the ICC structures of the artificial bone marrow constructs contemplated herein, are geometrically similar to the 3-D morphology of the inner part of bone supporting bone marrow tissue, which is important for creation of the microenvironmental niches that maintain stem cell survival and promote maturation. ICC topology is also convenient because it affords a simple method of control over cellular interactions and migration by varying the sphere diameter. In some embodiments, artificial bone-marrow constructs can be created in a tissue culture plates having one or more wells of any desirable dimension.

The bone marrow construct can comprise an ICC scaffold made from suitable biocompatible materials, for example, a hydrogel material or an inorganic and/or organic construct containing silicon and silicate materials. The ICC scaffold design described in detail above can have spherical voids or cavities with diameters ranging from about 5 to about 200 µm, for example, or about 10 to about 200 µm, or about 90 to about 200 µm, or about 100 to about 200 µm, or about 125 to about 200 µm or about 150 to about 200 µm, or about 5 to about 150 µm, or about 10 to about 125 µm, or about 15 to about 100 µm, or from about 20 to about 75 µm. In some embodiments, the colloidal crystal voids or cavities are from about 5 to about 100 µm in diameter. The diameter of the spherical spaces or cavities are believed to provide efficient contacts between adhesion and dispersion cells and allows for natural cell migration through the inter-connecting pores between the microspherical voids, which can be important for replication of hematopoietic tissues.

In some embodiments, the ICC scaffold comprises a three dimensional polymer matrix including a transparent polymer network having a plurality of empty spherical cavities with interconnected pores arranged in a hexagonal crystal lattice. In some embodiments, the inter-connecting pores range in size from about 1 to about 100 µm, or from about 3 to about 100 µm, or from about 5 to about 100 µm, or from about 10 µm to about 100 µm, or from about 75 to about 100 µm, or from about 80 to about 2 µm, or from about 75 to about 2 µm, or from about 50 to about 2 µm, or from about 25 to about 2 µm. In some embodiments, the pore size can range from about 20 to about 2 µm.

In some embodiments of the present disclosure, the bone marrow construct comprises an ICC scaffold coated with one or more polyelectrolytes using the LBL method described herein. To provide adequate adhesion of bone marrow support cells or stromal support cells, a hydrogel matrix can be coated with clay and poly(diallyldimethyl ammonium chloride) multilayers following the LBL method described above which results in a thin layer of nanocomposite on the walls of the scaffold. Such coatings can enhance cell adhesion on native unmodified hydrogel surfaces.

In some embodiments, the nanoscale nature of the LBL coating can be advantageous for several reasons, for example, (i) the hybrid organic-inorganic composite is mechanically compatible with the hydrogel and does not delaminate; (ii) it has high Young modulus necessary for successful cell adhesion; and (iii) nanocomposites have minimal light scattering because the characteristic diameter of inorganic component is smaller than the wavelength of light, which is quite relevant for optical interrogation of biological processes and markedly differentiates ICC hydrogel scaffolds from those previously used for bone marrow cultures.

The LBL coated ICC scaffolds made from transparent hydrogel material or silicates can then be functionalized with one or more bioactive agents to induce the differentiation, growth and maintenance of hematopoietic stem cells (HSCs). The one or more bioactive agents can be applied onto the spaces and pores of the ICC scaffold or alternatively, the bioactive agent can added to the culture medium supporting the cells of the construct. In some embodiments, the bioactive agent can be applied in any manner commonly known, for example, the bioactive agent can be applied to the walls and surfaces of the spaces and pores using spraying, dipping, suctioning, injecting techniques commonly used to apply a biological agent to a solid surface.

In some embodiments, the LBL coated ICC scaffolds can be populated with bone marrow cells which can include, for example, stem cells, stromal cells, immune cells and red blood cells and combinations of these. Generally, a bone marrow cell can be any cell found in mature and/or immature bone marrow from a mammalian subject, preferably human, but can also include the bone marrow of laboratory animals, domesticated animals, farm animals, and exotic animals. Bone marrow cells can in some embodiments, be a pure population of any one cell type found in mature or immature bone marrow or a population of a mixture of two or more of these cell types. In some embodiments, stem cells, preferably, HSCs (CD34+ HSCs and/or CD150+ HSC and/or CD 133+ HSC) are capable of differentiating into a variety of cell types. Illustratively, the stem cells, including, HSCs, can be used to construct the artificial bone marrow construct. The HSCs can be manipulated to differentiate into hematopoietic and non-hematopoietic cell types, including for example, cells of the erythrocyte and B- and T- cell lineage. In some embodiments, the autogeneic, allogeneic or xenogeneic HSCs can be induced to differentiate into B-cells and T-cells by applying one or more bioactive agents, for example, bioactive agents comprising: IL-2 (1-50 ng/mL, Calbiochem), IL-7 (1-200 ng/mL, R&D Systems), Flt3 ligand (1-200ng/mL, Chemicon), stem cell factor-1 (SDF-1) (1-200 ng/mL, Calbiochem), BMP-4 (1-100 ng/mL R&D Systems), and interleukin 3 (IL-3) (1-100 ng/mL, Chemicon). Additives used to promote development of a B lymphocyte lineage can also include: soluble CD40L (1-100 ng/mL, Invitrogen), IL-4 (1-100 ng/mL, Calbiochem), IL-5 (1-100 ng/mL, Chemicon), IL-6 (1-100 ng/mL, Calbiochem), IL-10 (1-100 ng/mL, Chemicon), IL-2 (1-100 ng/mL, Calbiochem), IL-7 (1-200 ng/mL), FLt3 ligand (1-200 ng/mL), stem cell factor (SDF) (1-200 ng/mL), interleukin 3 (IL-3) (1-100 ng/mL) and 1-500 µg/mL agonist anti-CD40 mAb (clone HM40-3; BD Biosciences). In some embodiments cell cultures having mature and undifferentiated immune cells (for example, T-cells, B-cells, Natural Killer cells (NK cells), granulocytes, dendritic cells, macrophages and monocytes) can be exposed to 0.01 to 10 µg bacterial lipopolysaccharide (LPS) (final concentration in the cell culture: 0.1 - 50 µg/mL LPS) commercially available, for example, Cat. No. L5668, Lipopolysaccharides from *Escherichia coli* 0127:B8 (1mg/mL, 0.2µm filtered) from Sigma-Aldrich, (St. Louis, MO, USA) to simulate T-independent signals. As used herein the concentration of the bioactive agents are expressed as the final concentration of the bioactive agent in the ICC scaffold. The cells may be cultured in the ICC scaffolds for a period of 15 days to about 60 days to induce the proper phenotype desired.

In some embodiments, an artificial bone marrow construct can be obtained after about 3 to about 6 weeks of culturing the initial cultured cells with the some cell growth and differentiation factors described above. A population of immature CD 34+ HSC and CD 150+ HSC along with stromal cells and differentiated leukocytes and other bone marrow cells would indicate that a functioning artificial bone marrow construct has been achieved. The tailoring of specific cell factors to induce the expression and differentiation of one or more cell types can also signal a mature bone marrow construct. In some embodiments, the artificial bone marrow construct will replicate the natural numbers of one or more cell types found in a subject's bone marrow, and thus indicate that a natural and mature bone marrow phenotype has been created. Upon creation of a mature or desired artificial bone marrow construct, the formulation of the cell culture liquid containing the some cell growth and differentiation factors can be altered to maintain the cell diversity rather than to induce a population of a specific cell type, for example, by maintaining the "normal" bone marrow cell phenotype by using the host's serum factors in a ratio and concentration that would be equivalent to bovine serum as an illustration. In still other embodiments, once a mature bone marrow construct has been achieved, the expression and growth of one or more cell types, for example, B-cells and/or T-cells can be skewed by adding one or more commonly known B-cell and/or T-cell growth, differentiation and/or chemokine or chemoattractant factor(s) to drive the production or attraction of these desired mature or progenitor cells within the artificial bone marrow construct.

### Antigen Testing and Vaccine Development

In some embodiments, the present bone marrow construct can be engineered to produce an artificial immune network comprising B-cells and T-cells at some degrees of maturation and/or differentiation. In some embodiments, the artificial immune network comprises an ICC scaffold coated with one or more polyelectrolytes using the LBL method described herein. An illustrative example includes a hydrogel matrix or silicate matrix comprising ICC scaffolds having a plurality of voids or spaces, each space or void interconnected with one or more spaces or voids through pores as described above. In some embodiments, the matrix can be coated with clay and poly(diallyldimethyl ammonium chloride) multilayers following the LBL method described above. The artificial immune network can have a plurality of cell types including cells derived from hematopoiesis in general, and can include cells derived from lymphocytopoiesis, myelopoiesis and erythropoiesis. The combination of a bone marrow infrastructure complete with undifferentiated and terminally differentiated immune cells and appropriate growth and differentiation signals, provide a functional artificial immune network having one or more cell types, for example, immune cells comprising T-cells, B-cells, hematopoietic stem cells, stromal cells, lymphoid progenitor cells, plasma cells, monocytes, macrophages, natural killer cells, dendritic cells, neutrophils, eosinophils, basophils, mast cells, erythrocytes and at different maturation stages. Other non-lymphoid or blood cells may also participate in the support and maintenance of stem cells, stromal cells, T-cells and B-cells of the artificial immune network, including for example non-hematopoietic cells such as fibroblasts, epithelial cells, nerve cells, reticular cells, adipocytes and osteoid cells.

In some embodiments, the artificial immune network can be functionalized to produce a predominantly B-cell or antibody producing artificial immune network. As such, a B-cell artificial immune network can comprise hematopoietic stem cells and progenitor B-cells (at different levels of maturation and differentiation) and stromal cells. In some embodiments, the artificial immune network can include, HSC, CD34+ stem cells and stromal or feeder cells in the presence of appropriate growth and differentiation factors known to drive the expansion of CD34+ HSC and differentiation of B-lymphocytes to produce pro-B-cells, pre-B-cells, mature but antigen-naive B-cells, and plasma B-cells expressing IgM and IgG immunoglobulins and T-cells in some stages of immunological maturity. In some embodiments, the production of antibody producing B-cells can be observed by expression and measurement of key signal proteins and class switch of B-cells expressing IgM to IgG, secretion of antigen specific antibody, and when the artificial immune network is implanted *in-vivo,* production of human immune cells. In some embodiments, upon vaccination or antigen exposure, the B-cell artificial immune network resembles a primary and/or secondary lymphoid tissue such as follicles having one or more germinal centers.

In some embodiments, the artificial immune network can be developed to express antibody to a desired antigen *in-vitro* and/or *in-vivo* when implanted into an animal subject for example a mammalian subject such as a human subject, and veterinary subject such as a dog, cat, horse and the like or for experimental use in laboratory animals commonly employed in testing the effects of antibodies. Generally, any antigen can be used to prime the expression of antigen specific antibodies.

As used herein, "antigen(s)" and "immunogen(s)" are used interchangeably, and can refer to a compound which may be composed of organic molecules, for example, amino acids, carbohydrates, nucleic acids or lipids individually or in any combination. In some embodiments, the antigen can include any peptide, polypeptide, or derivative thereof or protein for which an immune response or antibody production is desired. These include, but are not limited to, peptides, polypeptides, proteins and derivatives thereof, such as glycopeptides, phosphopeptides, peptide nucleic acids and the like.

The term coupled antigens as used herein, refers to a compound which binds to one or more proteins and which is representative of the immunogen toward which an immune response is desirably directed. For example, where the immunogen is an influenza virus, the coupled antigen can include a peptide fragment of the matrix protein of the influenza virus. Immunogens can include antigens from neoplastic cell, infected cell, pathogen, or component thereof, towards which an *immune response* is to be elicited. In particular, the antigenic domain of the coupled antigen is selected to elicit an immune response to a particular disease or pathogen, including peptides obtained from MHC molecules, mutated DNA gene products, and direct DNA products such as those obtained from tumor cells.

Synthetic peptide and polypeptide derivatives or analogs, or any other similar compound that can be conjugated to another polypeptide or protein (i.e. a carrier protein) can be used in the present disclosure. Moreover, these peptides, proteins and derivatives may comprise single epitopes or multiple epitopes for generating different types of immune responses. Indeed, if an entire protein is used as an antigen, this protein is likely to comprise numerous epitopes, which may vary depending upon the solvent conditions and their effect on secondary and tertiary structure of the protein. Carbohydrates, nucleic acids and other non-protein substances also may be used as the antigenic moiety. Methods are available in the art for conjugating these substances to peptides or proteins to enhance immunogenicity. Some common coupling proteins for use as carrier proteins for antibody production can include bovine serum albumin, transferrin, and other large molecular weight proteins. Most coupling methods rely on the reactive functional groups in amino acids, such as -NH₂, -COOH, -SH, and phenolic -OH. Site-directed coupling is preferred over random coupling. Another peptide method used in anti-peptide antibody production is the Multiple Antigenic Peptide system (MAPs). The advantage of MAPs is that the conjugation method is not necessary. No carrier protein or linkage bond is introduced into the immunized host. In some embodiments, the small non-immunogenic molecules, including specific sequences of amino acids can be coupled to other complex structures including lipophilic adjuvants as described in "Synthesis of a Highly Pure Lipid Core Peptide Based Self-Adjuvanting Triepitopic Group A Streptococcal Vaccine, and Subsequent Immunological Evaluation" Moyle, P.M. et al., Med. Chem., (2006), Vol. 49 (21), 6364-6370, 2006. In some embodiments, the selected antigen can be made immunogenic using a variety of known coupling systems and adjuvants.

Other substances that can be used as the antigen and especially when coupled to a larger polypeptide or lipid molecule can include: small molecules, such as (1) metabolic byproducts (especially those that are toxic); (2) some environmental toxins or irritants (e.g., aromatic hydrocarbons, asbestos, mercury compounds and the like); (3) drugs (e.g., neural stimulants and opioids, for example, cocaine, amphetamines, ecstasy, heroin, nicotine, etc.) for treating addiction; and (4) venoms from snakes, spiders or other organisms. Many of these kinds of small molecules are non-antigenic or weakly antigenic, so would be appropriate candidates for use in the present disclosure. In some embodiments of the present disclosure, the antigen can include agents that are weakly antigenic or non-antigenic under currently available immunization conditions. Many tumor-associated antigens fall into this category, because the antigens also are expressed by normal cells. Therefore, immunological tolerance to such molecules makes it difficult to stimulate responses against such antigens. Other proteins that fall into this category include naturally occurring proteins from one species (e.g., human) for which it would be desirable to produce antibodies in another species but which are recalcitrant to antibody generation in the other species.

While the present disclosure may be applied to any type of antigen and/or immunogen, immunogens of particular interest are those associated with, derived from, or predicted to be associated with a neoplastic disease, including but not limited to a sarcoma, a lymphoma, a leukemia, or a carcinoma, and in particular, with melanoma, carcinoma of the breast, carcinoma of the prostate, ovarian carcinoma, carcinoma of the cervix, colon carcinoma, carcinoma of the lung, glioblastoma, astrocytoma, etc. Selections of melanoma antigens useful in hybrid antigens of the present disclosure may be found, by way of non-limiting example, in PCT/US01/12449 (WO0178655). Further, mutations of tumor suppressor gene products such as p53, or oncogene products such as the Harvey Ras oncogene may also provide coupled antigens to be used according to the present disclosure.

In some embodiments, the selection of the cancer antigen for inclusion into the artificial immune network for developing cancer specific antibodies and cancer specific immune cells (including CD4 and CD8 T-cells, NK cells, and antibody producing B-cells) for use as a cancer therapeutic can be accomplished in several ways known to those in the immunological arts. For example, antigens that are recognized *in-vivo* by human B or T cells can be identified by a number of techniques, which include probing extracts of laboratory or clinical tumor isolates with antibodies or T cells obtained from cancer patients and then identifying the individual antigens within the extract that are recognized by these immune probes. Alternatively, the selection of specific cancer antigens can be performed rapidly and generally in high-throughput using molecular techniques such as SEREX analysis or phage libraries. Although these approaches identify molecules that are antigens (they are recognized by antibodies or T cells). In some embodiments, a preferred method of antigen selection can include identifying antigens that are immunogenic in humans by immunize persons with the isolated non-cancer forming antigen(s) in question and determine whether a B- or T-cell response has been induced. This approach has been used to identify MAGE series of antigens, including for example, MART-1/melanA, gp100, tyrosinase, the gangliosides GD2, O-acetylated GD-3 and GM-2, and a urinary tumor-associated antigens. Methods for identifying and selecting cancer antigens useful in the present methods can be found for example in Euhus DM, et al., (1990) Cancer Immunol. Immunother.; 32: 214-220 and Livingston P. (1993) Ann. N. Y. Acad. Sci., 690: 204-213.

In some embodiments, methods for selecting and identifying antigens which can be incorporated into the artificial immune network for the development of B-cell and T-cells responses (*i.e.* immune cells capable of targeting cancer cells or capable of producing cancer antigen specific antibodies) to specific cancer antigens can include vaccine-induced immune response (VIIR) analysis. Subjects, including human and animal subjects can be immunized with polyvalent vaccines that contain multiple potential cancer cell immunogens and determine which stimulate immune responses *in-vivo* by comparing the profile of antibody and/or T-cell responses to antigens in the vaccine before and after vaccine treatment. The procedure can be iterated by immunizing individual antigens that are included in the selected vaccine to identify purified antigens capable of stimulate immune responses *in-vivo.* In some embodiments, the polyvalent antigens can comprise tumor specific cell-surface antigens. Bystryn JC, Shapiro RL, Oratz R. Cancer vaccines: clinical applications: partially purified tumor antigen vaccines. In: DeVita V, Hellman S, Rosenberg SA, eds. Biologic Therapy of Cancer. 2nd ed. Philadelphia, Pa: JB Lippincott; 1995:668-679 describes several approaches for the selection of tumor cell antigens that are useful in the present disclosure.

Antigens capable of inducing T-cell and/or B-cell responses *in-vivo* can be coated on the walls of the ICC scaffolds or may be administered into the artificial immune network either as solubulized antigen or complexed and/or coupled antigens as described above. In some embodiments, the amount of antigen or complexed/coupled antigen added to the ICC scaffolds can range from about 1ng to about 1 mg depending on several factors, for example, on the antigen tested, the immunogenicity of the antigen and the size of the ICC scaffold. In some embodiments, the cancer antigens are complexed in the form of linked molecules or as part of dendrimer molecules. The pure antigen or mixtures of cancer cell antigens used to prime and stimulate populations of immune cells growing in the artificial immune networks to produce plasma B-cells and primed T-cells, are capable of producing cancer antigen specific immune responses and in particular antibodies that are cancer cell specific for inclusion into therapeutic cancer compositions.

In some embodiments, the immunogen may be associated with an infectious disease, and, as such, may be a bacterium, virus, protozoan, mycoplasma, fungus, yeast, parasite, or prion. For example, but not by way of limitation, the immunogen may be a the entire organism or one or more cell surface antigens of a human papilloma virus (see below), a herpes virus such as herpes simplex or herpes zoster, a retrovirus such as human immunodeficiency virus 1 or 2, a hepatitis virus, an influenza virus, a rhinovirus, respiratory syncytial virus, cytomegalovirus, adenovirus, Mycoplasma pneumoniae, a bacterium of the genus Salmonella, Staphylococcus, Streptococcus, Enterococcus, Clostridium, Escherichia, Klebsiella, Vibrio, Mycobacterium, amoeba, a malarial parasite, *Trypanosoma cruzi,* etc.

Immunogens may be obtained by isolation directly from an infectious organism, an infected cell, a specimen from an infected subject, a cell culture, or an organism culture, or may be synthesized by chemical or recombinant techniques. In some embodiments, suitable antigenic peptides, for use against viruses, bacteria and fungi and the like can be designed by searching through their sequences for MHC class I restricted peptide epitopes containing HLA binding sequences such as but not limited to HLA-A2 peptide binding sequences. Although the size of the antigen may vary, the antigen may be the size of a polypeptide having between 10 and 500 amino acid residues. In some embodiments, the antigen can be a peptide having between 14 and 100 amino acid residues. In some embodiments, the specific antigen can be a fragment of a larger antigen coupled to another larger protein to serve as the antigenic domain, or, alternatively, to synthesize a peptide antigen by chemical or recombinant DNA methods.

In some embodiments of the present disclosure, the infectious antigen selected can be added to the artificial immune network containing cultures of stem cells, for example, human cord blood derived CD34⁺ HSCs. Cultures can be primed to proliferate and differentiate into mature B-cells and thereafter, the B-cell population can be expanded using anti-IgM crosslinking on day 14 of culture. The primed B-cells and other immune function cells in the network can then be exposed to one or more infectious organism antigens at concentrations ranging from about 0.001 µg to about 1.0 mg/ml of culture fluid on days 20-40 of culture. Cultures are capable of producing mature IgG expressing cells after 40 days of culture as analyzed by confocal microscopy or flow cytometry. The immune cells thus produced in the artificial immune network, can be used as a therapeutic by administering the cells and/or antibodies directed to a desired antigen or antigen complex to a subject in need of such therapy, for example as when the subject has a specific infectious agent or a specific tumor to which the immune cells have been targeted.

In some embodiments, the present disclosure also provides for implanting artificial immune networks into subjects with a cancerous or infectious disease. The artificial immune networks can be prepared using autogeneic or allogeneic stem cells *ex-vivo* using for example, autogeneic stem cells, including HSCs derived from the subject's blood, bone marrow, cord blood, adipose tissue and skin follicles. In some embodiments, allogeneic stem cells, including human cord blood derived CD34⁺ HSCs can be used to generate primed B-cells and/or T-cells and other immune function cells against an isolated cancer or infectious agent antigen present in the artificial immune network. In some embodiments, the disease specific antigen is isolated from the subject prior to implanting the artificial immune network to ensure that the immunological response is directed to the particular cancer or infectious agent present in the subject. Once the artificial immune network contains a sufficient level B-cells and /or T-cells producing disease specific antibodies and/or disease specific T-cells, the artificial immune network can be removed from the substrate *in-vitro* (for example a tissue culture plate or Petri dish) and implanted into the subject at a specific site, for example, intraperitoneally. In some embodiments, the artificial immune network can be implanted at or near the site of the tumor or infection.

### Production of Universal Blood

In some embodiments, the artificial bone marrow construct can be used to produce a universal blood product, containing primarily mature erythrocytes. Erythropoiesis is the development of mature red blood cells (erythrocytes). In some embodiments of the present disclosure, the artificial bone marrow constructs can be used to synthesize erythroblast and mature blood cells. The hematopoietic function of the artificial bone marrow can be directed to produce fully functioning red blood cells with any desired surface antigenic expression. In some embodiments, the red blood cells produced can be free of antigenic determinants of the ABO and Rh system to produce truly universal red blood cells having no A, B or Rh antigen.

The first cell that is recognizable as specifically leading down the red cell pathway is the proerythroblast or hemocytoblast. As development progresses, the nucleus becomes somewhat smaller and the cytoplasm becomes more basophilic, due to the presence of ribosomes. In this stage the cell is called a basophilic erythroblast. The cell will continue to become smaller throughout development. As the cell begins to produce hemoglobin, the cytoplasm attracts both basic and eosin stains, and is called a polychromatophillic erythroblast. The cytoplasm eventually becomes more eosinophilic, and the cell is called an orthochromatic erythroblast. This orthochromatic erythroblast will then extrude its nucleus and enter the circulation as a reticulocyte. Reticulocytes are so named because these cells contain reticular networks of polyribosomes. As reticulocytes loose their polyribosomes they become mature red blood cells.

The artificial bone marrow constructs containing HSC CD34⁺ stem cells and stromal support cells described above can be used to generate hemocytoblasts. Upon proper stimulus for both growth and differentiation signals, the development of mature red blood cells from the HSCs in the present bone marrow construct can driven by the hormone erythropoietin. Methods for deriving mature universal red blood cells from stem cells and red blood cell progenitor cells are well known in the art. The methods described in Douay, L. et al., (2007), Transfusion Medicine Reviews, 21(2): 91-100) are useful in the present disclosure to produce large numbers of mature red blood cells *ex-vivo* from hematopoietic stem cells obtained from blood, bone marrow and cord blood.

In some embodiments, the artificial bone marrow constructs described herein can be seeded with human or animal HSCs to produce species specific mature red blood cells using the species specific growth and differentiation factors required for red cell production without undue experiment. It is contemplated, that the use of the artificial bone marrow constructs described herein with methods of differentiating stem cells and red blood cell progenitor cells to terminally differentiated red blood cells can be employed to produce universal blood suitable for any mammal, including human, higher-primates, monkeys, laboratory animals, zoological animals, domesticated animals and exotic animals.

In some embodiments, the mature red cells can be treated with specific glucosidases which has been previously shown to remove A and B antigens on the red blood cell surface to render them universal. Methods and reagents for removing such RBC cell surface antigens are found in Liu QP, Sulzenbacher G, Yuan H, et. al. Bacterial glycosidases for the production of universal red blood cells. Nat. Bio-technol. (2007), 25: 454-464, which find utility in the present disclosure. In some embodiments, the enzymes responsible for removing the A and B cell surface antigens can be added to the artificial bone marrow construct during the production of mature red blood cells. The enzymes can be added to provide a final concentration of up to 0.5 mg/mL or alternatively can be incorporated into the surfaces of the ICC scaffold structure using some spraying and contact methods described above.

In some embodiments, the present methods and devices also enable the production of blood cells that are autologous to the patient in need of such cells. As described above for the production of universal red blood cells, stem cells whether they be embryonic, mesenchymal, or derived from the umbilical cord, or other somatic tissue such as bone marrow, adipose tissue, hair follicle and the like, can be differentiated and induced to be terminally differentiated into B-cells, T-cells, macrophages, monocytes, NK cells, and other lymphocytes, dendritic cells, neutrophils, basophils, eosinophills, platelets, mast cells and any desired blood cell type that can be differentiated from the above stem cells. In some embodiments, the methods used above to produce red blood cells can also be used to produce large numbers greater than about 10⁶ to about 10⁹ or greater numbers of stem cells including, for example, pluripotent stem cells, progenitor cells, partially differentiated stem cells and the like. In some embodiments, the present disclosure provides for the farming of hematopoietic stem cells and mesenchymal stem cells from autologous, allogeneic and xenogeneic sources. Such sources can include bone marrow, e.g. bone marrow aspirate, adipose tissue, for example derived from aspirated liposuction procedures and from tissue bank sources, e.g. whole blood, umbilical cord blood and the like.

### Possible variations and modifications

The dimensions of the ICC scaffold can be modified to fit microplates with different sized wells. For example, the vial size used to make colloidal crystals can be altered to fit the dimensions of 24-, 48-, 96-, 384, or 1536 well microplates, and ICC scaffolds fitting these microplates can be designed. This may be beneficial for studies, such as such as tissue engineering, requiring greater numbers of cells or longer cultures than allowed by a cell culture well microplate. Additionally, the dimensions can be modified to fit a perfusion bioreactor. This is particularly useful when not only are cells a final product, but also if a molecule produced by cells, such as an antibody or a hormone, is the desired product.

The materials of which ICC scaffolds are made can be changed according to a specific application. For example, a biodegradable polymer, such as PLGA or poly(e-caprolactone) can be substituted for hydrogel. Any material that is soluble in liquid, where consistently-sized microspheres that will not be dissolved by the same solvent exist, can be used to create an ICC scaffold. Also, it was mentioned in the technical description of the LBL process that the LBL coating materials can be altered.

Additionally, the present disclosure is also directed to a commercial kit comprising inverted crystal colloidal scaffolds sealed in a sterile package and instructions for use thereof in culturing cells. The scaffold can be included in a kit that includes a sterile polystyrene tissue culture plate with the standard number of wells 6, 12, 24, 48, 96 384 or 1536 wells within which the scaffolds have been placed, instructions for the cellular seeding and/or optimal dispersion concentration of growth/active factors, and accessory tools for proper scaffold handling. In a different approach, the present disclosure can feature a kit that includes sterile pre-formed three-dimensional scaffold shapes, a lyophilized or a combination of lyophilized growth/active factor(s), associated tools to allow the delivery of the lyophilized agents homogenously within the scaffold, and instructions for proper growth/active factor dispersion. In a different approach, the present disclosure can feature a kit that includes sterile pre-formed 3-D scaffold shapes, a lyophilized or a combination of lyophilized growth/active factor(s), a photopolymerizable agent, a vial to mix the photopolymerizable agent with the lyophilized compound, associated tools to allow the homogenous distribution of the photopolymerizable agent plus lyophilized compound into the scaffold, and necessary instructions. The kit could or could not include a light source to induce local photopolymerization, thus, trapping of the lyophilized compound into the 3-D scaffold.

The following examples describe embodiments within the scope of the claims herein, and other embodiments within the scope of the claims will be apparent to those skilled in the art from an understanding of the specification or practice of the disclosure as disclosed herein. It is intended that the specification along with the examples are to be considered as exemplary only, with the scope and spirit of the present disclosure being indicated by the claims. In the examples, all percentages are given on a weight basis unless otherwise indicated.

### EXAMPLES

### Example 1.

### Construction of ICC scaffolds for tissue growth and repair.

ICC scaffolds can be made with poly(lactic-co-glycolic acid) (PLA-PLGA) that has a lactic to glycolic acid ratio of 85:15. The co-polymerized polymer has a faster degradation rate than each of the single components, i.e. PLA or PLGA. They are very stable at room temperature when stored in dry format.

An upside-down beaker is placed into a sonication bath, and a 9 mm outer diameter vial is clamped on top of the beaker. A 9 in Pasteur pipette is clamped with its narrow end suspended inside the center of the vial. Teflon tape can be used to seal the opening of the vial and secure the pipette in the center, and the apparatus was filled with ethylene glycol. Soda lime spheres (Duke Scientific, Palo Alto, CA) with a diameter of 99.8 µm and size distribution of 3.2% are added to ethylene glycol in a dropping bottle. Under constant sonication, two drops of spheres are dropped through the pipette into the vial every fifteen minutes until the precipitated spheres have reached a desired height. The pipette is removed, and ethylene glycol is evaporated from the vial at 160 °C overnight. Spheres can be then heated at 675 °C for 3 h to anneal adjacent spheres into a solid colloidal crystal. The colloidal crystal is then infiltrated by submerging in 10% (w/v) 85:15 poly(lactic-co-glycolic acid) (PLGA), (Absorbable Polymers International, Pelham, AL) in dichloromethane, and sonicated for 3 hours or centrifuging at 5,900 rpm for 10 minutes. Infiltrated colloidal crystals are then placed into a vial with a small volume (to cover colloidal crystals) of 10% PLGA, and solvent is allowed to evaporate at room temperature overnight and under vacuum for an additional 24 hours. Soda lime beads can be removed from the composite colloidal crystal scaffold by stirring in 1% HF for 3 h, followed by rinsing several times with water. The resulting inverted colloidal crystal structure can then be examined by light microscopy for complete removal of soda lime beads. If beads are visible on the surface, a layer of PLGA can be scraped off the surface with a razor, and re-immersed in HF. This can repeated until all beads are removed.

PLA-PLGA has been known as a material for regenerative medicine, but in case of artificial skin, grafts with greater flexibility and ability to conform to the body curvatures are desired. Alginate can be used to make such scaffolds. Alginate is a biodegradable scaffolding material with the mechanical properties similar to that of hydrogel. The calcium alginate scaffolds can be prepared from high-G alginate and calcium chloride by the gelatin-freeze technique, which consists of the following steps: (1) preparation of 2% (w/v) sodium alginate stock solutions; (2) cross linking the alginate solution by adding an equal volume of calcium chloride solution (the final concentration of Ca²⁺ is 0.01 M), while stirring intensively using a homogenizer at 2,000 rpm for min; (3) transferring the sol-gel into a dish or into the colloidal crystal mold and freezing the cross-linked material, at -18°C, overnight; and (4) melting the frozen material at room temperature. After the removal of the microspheres, the resulting gel like sponges are cut into small pieces and can be sterilized using ethanol solution and stored in distilled water at 4°C until use.

LBL coating on PLGA scaffolds with collagen. To construct an epidermal supporting layer on the ICC scaffold, collagen can be coated onto the PLGA ICC scaffold via LBL assembly. Polyacrylic acid (PAA) polyelectrolyte is used as a counterpart for the LBL deposition. Due to the negatively charged nature of PLGA, the coating can be applied by alternate deposition of positively charged collagen (type I from calf skin, 0.2 mg/mL in 0.1 N acetic acid solution, pH=4) and oppositely charged PAA (1mg/mL, pH=3) onto the scaffold.

A Microlab STAR liquid handling system (USA) can be used to apply the coating automatically. The scaffold can first be placed into a well of microplate (48 well). 400 µL collagen solution is transferred into the well with a pipette programmed automatically and kept for 20 min for the deposition of collagen layer on scaffold. After the collagen deposition, the collagen solution is removed from the well for disposal. De-ionized (DI) water is then brought into the scaffold well to rinse the scaffold twice for 5 min (2.5 min each). Following the same procedure, 400 µL PAA can be transferred into the well, and the solution is left for 10 min for the PAA layer deposition, followed by D.I. water rinsing twice for 5 min after PAA solution is removed. The same cycle can be repeated until the desired layer numbers was obtained. In our preparation, the LBL coating can be carried out repetitively to achieve 37 bilayers of collagen/PAA [(collagen/PAA)₃₇] on PLGA scaffold. In order to estimate the coating layer thickness, structure and topography, the first and last layers of PAA are replaced by fluorescent-labeled trypsin inhibitor (10 µg/mL) which emits in green channel for confocal observation. The scaffold can be sectioned in order to inspect the cross section for the LBL coating. UV-Vis spectroscopy, transmission optical microscopy, confocal microscopy, atomic force microscopy, and SEM can be used to inspect the coated scaffold.

Degradation rate of PLGA ICC scaffold. The PLGA scaffold size can shrink about 25% over 2 weeks in PBS buffer (pH 7.4). This rate can be used for assessment of the degradation of the prepared scaffolds *in-vivo,* although we observed that the decay of ICC constructs in mice is significantly faster than in PBS buffer. The rate of scaffold can shrink slowed down after the first two weeks, which is possibly due to the degradation kinetics following an exponential decay pattern. This can be controlled by optimizing the architecture of the scaffold.

Biocompatibility of the ICC-LBL hybrid scaffolds. The biocompatibility of the ICC-LBL hybrid scaffolds with *in-vitro* cell cultures can be tested using ICC scaffolds having a functionalized matrix including voids surfaces and pores. The hybrid scaffolds can be pre-soaked with culture medium (DMEM with 20% FBS and 1% Pen-Strip) for 24 hours. Three mouse cell lines: epithelial XB-2, endothelial MS1 and fibroblast STO, can be seeded on the hybrid scaffolds that are placed in wells of a 48-well microplate, the number of cells per scaffold was 5x10⁴ for each line. Culture media can be changed every 48 hours. One week later the cells can be stained with a fluorescence viability kit commercially available from Molecular Probes Inc. (Eugene, OR USA) and can be inspected with a confocal microscope.

### Example 2.

### Artificial Bone Marrow Constructs.

Primary colloidal crystals are hexagonally packed lattices of spheres (FIGs. 7A - 7C), with a wide range of diameters from nanometers to micrometers. ICCs are similarly organized structures where the spheres are replaced with cavities, while the interstitial spaces are filled. Floating HSCs enter into a pore through interconnected channels that have diameters 2-5 times larger than that of a single cell. Temporarily entrapped HSCs undergo intense contacts with stromal cells residing on the pore surface. (FIGs. 7D - 7F). When ICC cavities exceed the diameter of cells, they can be used as 3-D cell scaffolds. The open geometry of the ICC lattice, high porosity (60% - 90% free space), full interconnectivity, and large surface area make ICC an attractive structure for studies of 3-D effects in cell cultures. It is also apparent that the ICC structure is geometrically similar to the 3-D morphology of supporting bone marrow tissue in a trabecular bone, which is important for creation of the microenvironmental niches that maintain stem cell survival and promote maturation. In addition, ICC topology is convenient because it affords a simple method of control over cellular interactions and migration by varying the sphere diameter. For FIGs. 7D and 7E, the diameter of the spherical cavities is approximately 110 µm, inter-connecting pores is ca. 15-25 µm. Lastly, dynamic culture within a rotary cell culture vessel was beneficial not only by creating more realistic physiological environment but also by maximizing the role of specifically designed ICC geometry by generating continuous convective media flow into ICC pores. (FIG. 7F)

Throughout this example, poly(styrene) beads with a diameter of 110 µm were used, producing the cavities of the same size connected by 10-25 µm channels (FIGs. 7A - 7C). This diameter was chosen because it provides efficient contacts between adhesion and dispersion cells, and allows for natural cell migration through the channels between the cavities, which is imperative for replication of hematopoietic tissues (FIGs. 7G - 7H). The materials of a typical ICC scaffolds used here can be silicate or hydrogels among others, for example, poly(acrylamide) hydrogel, both of which were demonstrated to be compatible with human cell culture. To provide adequate adhesion of bone marrow support cells, hydrogel matrixes were coated by clay and poly(diallyldimethyl ammonium chloride) multilayer following the layer-by-layer (LBL) technology, which results in a thin layer of nanocomposite on the walls of the scaffold. (See FIG. 7E). Such coatings facilitate cell adhesion, which is typically quite poor on native un-modified hydrogels. Moreover, the nanoscale nature of the coating is essential for several reasons: (i) The hybrid organic-inorganic composite is mechanically compatible with the hydrogel and does not delaminate; (ii) It provides high Young's modulus necessary for successful stromal cell adhesion and (iii) Nanocomposites have minimal light scattering because the characteristic diameter of the inorganic component is smaller than the wavelength of light, which is quite relevant for optical interrogation of biological processes. These features markedly differentiate ICC hydrogel scaffolds from those previously used for bone marrow cultures. In the framework of this study, no difference in silicate and hydrogel ICC scaffold performance in terms of bone marrow functionality was observed, although hydrogel scaffolds are preferred due to much higher transparency and ease of confocal microscopy examination.

Self-renewal of an undifferentiated population of CD34+ HSCs and production of fully functional immune cells of specific leukocyte lineages are the two basic functions of bone marrow most essential for applications mentioned above. Bone marrow stroma is comprised of a complex reticulum containing hematopoietic precursors, as well as non-hematopoietic cells such as fibroblasts, epithelial cells, nerve cells, reticular cells, adipocytes and osteoid cells. To mimic the bone marrow stroma tissue function, human bone marrow stromal cells were seeded on scaffolds and cultured for 3-7 days to allow the formation of a support cell layer on the scaffold surface prior to the addition of CD34+ HSCs (FIG. 8A). CD34+ HSCs were chosen because they have been shown to provide long term multi-lineage engraftment capability. CD34+ HSCs were isolated from human peripheral blood, umbilical cord blood or bone marrow. Adult CD34+ HSC were enriched by counter current centrifugal elutriation of peripheral blood MNL in a Beckmann J6M elutriator (Beckman Instruments, USA) using a Sanderson chamber. RPMI 1640 supplemented with 2 mM glutamine, 100 units penicillin G and 100 µg/ml streptomycin and 10% heat inactivated defined fetal calf serum (Hyclone, Utah) was used as elutriation medium. 3-6 X 10⁷ cells were loaded at 3000RPM and HSCs were isolated using a step-wise reduction of rotor speed until the appropriately sized cell population was isolated. Immuno-phenotypic analysis of elutriation with 5-7µ HSC at the time of isolation and seeding of the scaffold showed that these cells were negative or had very low expression of lineage specific markers CD117, CD135, CD 90, Lin1, CD45, HLA-Class I ABC, and HLA class II. These cells were negative for factor receptors EFGR, PDGFR, TGF-b, TNF R1, CD183 and CD123 and for expression of a wide variety of matrix receptor molecules including CD41, CD63 and CD9, which are platelet specific surface markers. CD34+ cells were then purified by negative selection of any remaining Lin-1 positive cells using Dynall magnetic beads.

All cells were positive for CD34 and were lineage-1 (lin-1) negative when seeded onto the scaffolds (FIG. 8B). A small portion (1-2%) of CD34-expressing cells was positive for CD150, a cell marker also associated with long term multi-cell lineage reconstitution in irradiated mice. Analogous cultures were also made on 2D plates to establish the importance of the 3-D geometry in ICC scaffolds. Examination of ICC cultures on day 14 showed the continued presence of CD34+ HSCs (FIG. 8C). There was also formation of numerous actin-rich cell processes (FIG. 8D), which were absent in cell cultures on flat substrates. Similarly, maintenance of a population of CD150+ cells (FIG. 8E) was seen in ICC matrices but not in donor matched 2D cultures after 28 days. Data from flow cytometry show that there were significantly higher percentages of CD34+ cells in ICC cultures after 28 days, regardless of the original cell source, when compared to 2D plate cultures (FIGs. 8F - 8K and 8L). This demonstrates that an undifferentiated population of CD34+ cells was maintained over time and demonstrates the importance of the chosen 3-D ICC organization of the cell cultures for replication of reproductive functionality of bone marrow. Active proliferation of HSC can also be seen from observation of mitotic figures (FIG. 8M) and analysis of loss of CFSE fluorescence intensity (FIGs. 8N - 8O). ICC scaffolds demonstrate substantially more CD34+ proliferation than plate cultures.

In some experiments, HS-5 human bone marrow stromal cells were seeded on the scaffolds and cultured for 3 days to allow formation of a dense layer on the scaffold surface (FIG. 8A) prior to the addition of CD34+ HSCs. CD34+ HSCs were chosen because they have been shown to provide long term multilineage engraftment capability. Analogous cultures were also made on 2D plates to establish the importance the 3-D geometry in ICC scaffolds. CD34+ HSCs were isolated from human peripheral blood, umbilical cord blood or bone marrow. FIGs. 8F, 8H and 8J show flow cytometry histograms depicting relative numbers of cells staining positive for CD34 derived from 3-D ICC scaffolds. FIGs. 8G, 8I and 8K show flow cytometry histograms depicting relative numbers of cells staining positive for CD34 positive cells derived from 2D plate cultures. The cells used in FIGs. 8F and 8G were obtained from bone marrow. The cells used in FIGs. 8H and 8I were obtained from cord blood. The cells used in FIGs. 8J and 8K were obtained from peripheral blood. 10,000 cytometry events were collected for all samples. Solid red histograms show CD34 levels with isotype controls for each sample shown as the green histogram overlay. All cells were positive for CD34 and Lin-1 negative when seeded onto the scaffolds; a small portion (1-2%) of low CD34 expressing cells was also positive for CD150, a cell marker associated with long term multi-cell lineage reconstitution in irradiated mice. Examination of ICC cultures on day 14 showed the continued presence of CD34+ HSCs. There was also formation of numerous actin-rich cell processes, which were absent in cell cultures on planar substrates. Similarly, a population of CD150+ cells could be seen in ICC matrices but not in donor matched 2D cultures after 28 days. Data from flow cytometry show that there were significantly higher percentages of CD34 expressing cells in ICC cultures after 28 days, regardless of the cell source, when compared to 2D plate cultures.

To assess the ability of the artificial bone marrow constructs to produce functional immune cells we focused on B lymphocyte production since B cells normally undergo the process of differentiation (as well as negative and positive selection) in the bone marrow. (J. Chen, Exp. Hematol. 33(8), 901-908, 2005; M. Punzel, Leukemia 13(1), 92-97. 1999; C. Civin, I et al. J. Clin. Oncol. 14(8), 2224-2233. 1996 and K. G. Murti et al. Exp. Cell Res. 226(1), 47-58. 1996).

Bone marrow is also the site of long term antibody production after viral infection and bone marrow stroma has been shown to play a role in plasma cell life cycle. Similarly to maintenance of HSC populations, the production of B cells is an essential component for development of *ex-vivo* bone marrow, such as immune system studies, development of human monoclonal antibodies, drug evaluation and disease treatment. B cell development involves a series of stages where close 3-D contact between bone marrow stroma and the developing B cell is critical and is hard to realize in 2D plate cultures. After 3 days of culture, ICC/stromal cell constructs containing growth factors to drive B cell production were seeded with CD34+ HSCs. To induce HSC differentiation, a co-culture of HS-5 bone marrow stromal, and H.fob 1.19 osteoblast cell line (ATCC) was initially used. Although differentiation was successful these cells lines quickly overgrew the matrix without irradiation and thus were somewhat inconvenient. Later, we created primary stromal support lines from bone marrow aspirates (Cambrex) which included marrow stromal cells positive for CD105 (100%), CD166 (100%), CD44 (95%), CD14 (1%), CD34 (1%) and CD45 (<1%) suggesting they were part of the stromal cell population; at least one cell type in the support cell mix was of osteoblast lineage as it was positive for osteonectin. These primary support cells formed densely populated layers similar to natural bone marrow and replicated the actual bone marrow environment better than the feeder layer made from a single cell type.

To induce HSC differentiation, a co-culture of HS-5 bone marrow stromal, and H.fob 1.19 osteoblast cell line (ATCC) was initially used. Although differentiation was successful these cells lines quickly overgrew the matrix without irradiation and thus were somewhat inconvenient. Later, we created primary stromal support lines from bone marrow aspirates (Cambrex) which included marrow stromal cells positive for CD105 (100%), CD166 (100%), CD44 (95%), CD14 (1%), CD34 (1%) and CD45 (<1%) suggesting they were part of the stromal cell population; at least one cell type in the support cell mix was of osteoblast lineage as it was positive for osteonectin. These primary support cells formed densely populated layers similar to natural bone marrow and replicated the actual bone marrow environment better than the feeder layer made from a single cell type. Growth factors used to promote hematopoiesis included: IL-2 (5ng/m, Calbiochem), IL-7 (20ng/ml, R&D Systems), Flt3 ligand (20ng/ml, Chemicon), stem cell factor-1 (SDF-1 20 ng/ml, Calbiochem), BMP-4 (R&D Systems), and interleukin 3 (IL-3) (10 ng/ml, Chemicon). Additives used to promote development of a B lymphocyte lineage included: soluble CD40L (5ng/ml, Invitrogen), IL-4 (10 ng/ml, Calbiochem), IL-5 (10 ng/ml, Chemicon), IL-6 (10 ng/ml, Calbiochem), IL-10 (10 ng/ml, Chemicon), IL-2 (5ng/ml, Calbiochem), IL-7 (20 ng/ml), FLt3 ligand (20ng/ml), stem cell factor (SDF 20 ng/ml), interleukin 3 (IL-3) (10 ng/ml) and 5 µg/ml agonist anti CD40 mAb (clone HM40-3; BD Biosciences).

Cell cultures were examined for stage-specific markers of development and functionality on days 1, 7, 14, 28 and 40. ICC cultures showed nuclear specific expression of Rag-1 by day 7 (FIG. 9A), cell surface IgM by day 14 (FIG. 9B), and co-expression of IgM with IgD (FIGs. 9C, 9D and 9E) by day 28 confirming differentiation of CD34+ into mature antigen naive B lymphocytes. In a separate comparative experiment, more of the differentiating cell population were seen to express CD40 (P=0.0002) and IgM/IgD co-expression (P= 0.021) in donor matched ICC cultures than in 2D cultures (FIG. 9F).

These results show the expression of phenotypic cell surface markers of B-cells, which is important step in development bone marrow replicas. However this fact does not necessarily prove the functionality of the *ex-vivo* generated B lymphocytes. To evaluate the ability of these B lymphocytes to respond to mitogenic or antigenic stimulation and fully mature into antibody producing cells, B lymphocytes isolated from 28 day ICC scaffold constructs and donor matched plate cultures were exposed to bacterial lipopolysaccharide (LPS), major structural component of the outer wall of gram-negative bacteria and the initiator of immune response to bacterial infection. Secreted IgM was quantified for all B cell cultures using a total human IgM ELISA assay (Diagnostic Automaton, Inc.) using CB-derived, PB-derived or BM-derived CD34+ cells induced to produce B cells. Significantly higher levels of IgM were produced from B-lymphocytes generated in the ICC scaffold regardless of the initial source of the CD34+ cells (FIG. 9G, notice difference in scales).

In a subset of experiments artificial bone marrow constructs were prepared as described above and were seeded with human cord blood derived CD34+ HSCs. Cultures were primed to proliferate and the B cell population was expanded using anti-IgM crosslinking on day 14 of culture. To expand B cell numbers, cells were stimulated by IgM crosslinking using Anti-IgM Affibody antibody (ABCAM). The antibody molecule was diluted to 4µg/ml and ICC Scaffolds were incubated in the antibody solution at 4°C overnight. The scaffolds were removed from the antibody solution and were washed three times in deionized distilled water and then three times in saline.

Cultures were then exposed to heat-killed whole influenza A virus (multiplicity if infection or MOI of 10) on days 28-30 of culture. Cultures yielded mature IgG expressing cells after 40 days of culture as analyzed by confocal microscopy (FIG. 9H) or flow cytometry (FIG. 9I) with an average production of 13.5 +/-9.4% IgM expressing (with no expression of IgD) and 3.1 +/-1.9 % IgG expressing cells (6 experiments), while isotype controls for all experiments were </=0.2% (6 experiments). Examination of influenza A antigen specific antibody production by hemagglutinin inhibition assay (HAI), neutralizing antibody assay titer, anti-HA IgG antibody ELISA, and anti-nuclear protein (NP) ELISA (5 experiments each) showed consistent production of specific antibody in all ICC scaffold cultures but never in donor matched 2D cultures receiving the same treatments.

**Table 1, Evaluation of specific influenza A/Caledonia antibody production by hemagglutinin inhibition assay (HAI), neutralizing antibody assay titer, anti-HA IgG antibody ELISA, and anti-nuclear protein (NP) ELISA (5 experiments each).**

| | Hemagglutinin Inhibition Assay | Neutralizing Antibody Assay Titer | Anti-HA ELISA (ng/ml) | Anti-NP ELISA (ng/ml) |
|---|---|---|---|---|
| 1 | 1/4 | 1/8 | 0 | 0 |
| 2 | 1/8 | 1/16 | 10 | 10 |
| 3 | 1/8 | 1/16 | 10 | 12 |
| 4 | 1/4 | 1/4 | 8 | 2 |
| 5 | 1/4 | 1/8 | 2 | 6 |

Results in Table 1 are arranged according to the highest dilution showing inhibition in case of HAI and neutralizing antibody or ng/ml concentrations of corresponding proteins in case of anti-HA and NP ELISAs.

ICC scaffold/stromal cell constructs seeded with CFSE-labeled cord blood derived CD34+ HSCs were cultured *in-vitro* for about 3 to 7 and then implanted on the backs of eight SCID mice to test their *in-vivo* functionality. The animals were sacrificed after 2 weeks and then the implanted bone marrow construct (FIG. 10A), peripheral blood and spleens of animals were collected for leukocyte subset pheno-typing. The regions near the construct were highly vascularized (FIG. 10A) although few red blood cells were seen in the construct itself. Flow cytometric evaluation of cells obtained from the construct, peripheral blood or spleens and confocal evaluation of the bone marrow construct itself showed that the majority of cells in the ICC construct were human major histocompatibility complex (MHC) class I+ (FIGs. 10B and 10H) and subsets of HSCs including CD34+ (FIGs. 10C and 10H), CD150+ (FIGs. 10D and 10H) and CD133+ (FIGs. 10E and 10H) were maintained. CD133+ and CD150+ are HSCs cell types that have been shown along with CD34+ cells to function in the repopulation of leukocyte *in-vivo.* High levels of CD19+ B lymphocytes (FIGs. 10F and 10H) and IgM expressing cells (FIGs. 10G and 10H) as well as B cell precursors (FIG. 10H) were also seen.

Examination of the cells populating in the spleen and peripheral blood showed that the majority of leukocytes were of human origin as indicated by MHC class I staining (FIG. 10H) and that the predominant cell type found was CD19+ positive (FIG. 10H). In the spleens of mice receiving implants most of the CD19+ cells were shown to co-express IgM and IgD (FIG. 10H). Median engraftment time for bone marrow reconstitution by transplant of bone marrow or cord blood is typically between 10-30 days. One can suggest that engraftment of HSCs in the artificial bone marrow construct of the present disclosure with continued production of CD34+ cells is similar to that seen in human or murine reconstitution of irradiated marrow. The level of cells was surprisingly high after engraftment of the bone marrow surrogate possibly because it is a better system overall than some used before and supports not only production of mature cells types but production of immature precursor cells at levels closer to that found in normal bone marrow. As seen before, there were high levels of CD34+ HSC replication, which could be maintained for an extended period of time.

The data obtained show that (i) proper organization of cells provide by the ICC scaffold has tremendous importance in the functionality of the *ex-vivo* replica, (ii) the described bone marrow construct replicates two of the key reproductive functions of normal bone marrow. This cell-scaffold system allowed for the growth, differentiation and movement of cells in a three-dimensional environment effectively mimicking the natural *in-vivo* bone marrow environment normally encountered during hematopoiesis/lymphopoiesis.

### Example 3.

### Differentiation of Stem Cells Into Selected Tissue Types

Mouse embryonic stem cell growth and differentiation can be analyzed using refined 3-D ICC scaffolds, co-cultured with selected skin cell lines (such as epithelial XB2, endothelial MS1 and fibroblast STO). The C57BL6 strain of murine ESCs can be divided into equal aliquots of 0.5-1X 10⁵ cells and can be seeded into ICC scaffolds of different geometries made previously and that will be placed in a multi-well microplate. Appropriate combinations of growth factors can be added in order to induce the cells to differentiate towards a smooth muscle, neural, chondrogenic or adipose lineage. For the differentiation of cells to a smooth muscle lineage: DMEM/ F12 w/ 10% fetal calf serum, and 3% human serum will be added. For differentiation to a neural lineage: DMEM/F12 w/ 10% FBS, 10% fetal calf serum, and β - mercaptoethanol (β-ME) can be added. If necessary, for development of the chondrogenic lineage serum free DMEM/F12 + ITS + premix and TGF- β1 can be used. For the development of an lineage, DMEM w/ 10% fetal calf serum, Dexamethasone and indomethacin can be added to the culture. The cells will be allowed to incubate after nine days of culture at 37° C in 5% CO₂. The wells will be evaluated for development and expression of lineage specific differentiation markers. Growth factors used to produce multiple cell lineages are as follows:

Adipogenic Medium: DMEM w/ 10% fetal calf serum, 50 µg/ml ascorbate-2 phosphate, 10 ⁻⁷M dexamethasome, 50 µg/ml indomethacin. Chondrogenic Medium: serum free DME/ F12 + ITS + premix and 10 ng/ml TGF- β₁. Neural Medium: DMEM/F12 w/ 10% FBS, 10% fetal calf serum, 5 X 10 ⁻⁷M β-mercaptoethanol (β - 2-ME), 10⁻³ M trans retinoic acid and 10% neural basal media (Cambrex). Smooth Muscle Medium: DMEM/F12 w/ 10% fetal calf serum. Fibroblast Medium. DMEM w/ 10% fetal calf serum, EGF, FGF and 10 ng/ml TGF-β₁.

In addition, the ICC scaffolds can be used to test co-cultures of the ESCs with skin-relevant cell lines including epithelial, endothelial, fibroblast and astrocytes, and observe the differentiation induced by the presence of these cells. When performing co-culture, the ESCs or the co-cultured cells will be stained with calcein or CFSE so that they are distinct from each other under confocal or fluorescence microscopy. The culture will be inspected using an in-house Nikon inverted fluorescence microscope daily to trace the CFSE stained cell, and also be fixed in 2% paraformaldehyde for confocal microscopy analysis. Scanning electron microscopy analysis can be used to assess the cells.

The ESCs can be analyzed for specific markers of cell differentiation for each cell lineage evaluated. Adipogenic development is determined after staining of cells with a dye Oil Red O. Chondrogenic development is evaluated using Safranin O histochemical analysis or immunocytochemical staining for type II collagen. Smooth muscle development is determined after immunohistochemical staining for anti-human alpha-actin. Neuronal development is determined after immunohistochemical staining for anti human nestin, alpha-tubulin and neuron specific nuclear protein.

Skin healing using stem cells and ESCAS is an important part of the step on this pathway will be the transfer of the differentiation procedures to human skin stem cells (HSSC). HSSCs will be extracted from the burn tissue as well as from the tissues left from cosmetic surgeries (face lifts, tummy tucks, etc) performed in any surgical suite. Skin derived stem cells similarly to ESCs can be much more suitable for the treatment of vesicant injuries because they provide both epidermal as well as dermal components (sweat glands, hair, fat, etc). This can potentially reduce or eliminate the disfiguring scarring occurring in most chemical or thermal burns. It is also envisioned that the use of HSSCs in skin repair can help form a more natural and functional skin tissue with most of the skin's components in place. This differentiates HSSCs from keratinocytes that can be purchased to achieve the same goal. The latter, however, represents only epidermis and these cells are not sufficient for the regeneration of the fully functional skin.

### Example 4.

### Effects of Methotrexate And Erythropoietin On Cell Function

Observation of the effect of currently used drugs on the constructed bone marrow replica can be used to validate the entire concept of drug testing *ex-vivo* and will make possible, the development of a standard protocol for the evaluation of specific activity of drug candidates. It will also provide technological foundation for the manufacturing of *ex-vivo* testing kits for pharmaceutical industry. The drugs, which are known to result in up- and down- regulation of bone marrow in humans, are hypothesized to produce a similar effect in *ex-vivo* replicas of bone marrow. Two drugs with well characterized effect on the bone marrow including Methotrexate (MT) (also known as Amethopterin, Rheumatrex, Trexall) and erythropoietin (EPO) (also known as Aranesp, Eprex, and NeoRecormon; similar drugs also include CERA and Dynepo. MT is as an antimetabolite drug and can be considered as a representative of a large class of anticancer drugs with similar action against rapidly dividing cells. It is also used in treatment of autoimmune deceases such as psoriasis and rheumatoid arthritis. It is known to inhibit the bone marrow function and, most likely, replication of CD34+ cells. In fact, most of chemotherapy drugs has inhibitory side effect on bone marrow, and therefore, testing with *ex-vivo* bone marrow can be one of the key tests in drug development.

Another drug/medicament, EPO, is a cytokine for erythrocyte precursors in the bone marrow. It is produced in the kidneys, and is used a therapeutic agent in treating anemia resulting from chronic kidney failure, cancer and other diseases. EPO up-regulates bone marrow function boosting the production of hematopoietic cells and, in particular, HSCs.

Testing of down-regulatory effect of MT on *ex-vivo* bone marrow constructs. Bone marrow constructs can be subjected to some concentrations of MT. Based on the clinical dosage of MT for adult patients, i.e. 5 - 15 mg. In the present analyses, 0-200 ng of MT per well are used in these experiments. Bone marrow replicas will be incubated to reach a confluent cell layer on the scaffold and after that, will be exposed to 0, 10, 50, 75, 100, 150, 200 ng of MT per well. Each experiment will be repeated 7 times to accumulate sufficient statistical information. The population of the cells in each well will be assessed on Day 1, 2, 3, 4, 5, 7, and 10 after addition of MT. The cells will be analyzed in terms of total cell count, which will provide important information on hematopoietic functions of the bone marrow replica. The population of the cells with the following markers will be assessed: CD34+, CD10, CD19, CD21, CD1a, CD3, CD4, CD8, CD 36, CD47, CD71 and IgM. Other cluster of differentiation molecules (CD) will be tested as well. The drop of cell count with CD34+ markers will indicate inhibition of HSC reproduction by MT. CD10 and CD1α will be used to identify B cell and T cell precursors, respectively. CD19, CD21 and IgM will aid in the understanding of the effect of MT on differentiation of CD34+ HSCs into B-cells. CD3, CD4 (helper T-cells), and CD8 (cytotoxic T cells) will distinguish the produced T-cells (if any). The data on the overall cell count and cell count for each marker is to be compared to the blank experiments and correlated with the amount of MT added. The correlation function is established; the threshold of significance will be considered to be that with r value equal or above 0.65 (r=1 is the perfect correlation).

Testing of up-regulatory effect of EPO on *ex-vivo* bone marrow constructs. Evaluation of effect of EPO on the functionality of the *ex-vivo* bone constructs will follow the same protocol as for MT. Bone marrow replicas obtained as a result of culturing hematopoietic cells in hydrogel ICC cell scaffolds will be subjected to some amounts of EPO. Each experiment will be repeated 7 times and the population of the cells with different amounts of EPO will be assessed on Day 1, 2, 3, 4, 5, 7, and 10 after addition of EPO. Similar correlations analysis between the concentration of EPO and the total cell output as well as flow cytometry cell counts for CD34+, CD10, CD19, CD21, CD1α, CD3, CD4, CD8, CD 36, CD47, CD71, IgM, and other markers can be established. A substantial increase in specific blood cells and overall acceleration of cell proliferation in bone marrow replicas is expected.

### Example 5.

### Functional Liver Tissue Constitution For In-Vitro Toxicology Screening Of New Drug Compounds.

ICC scaffolds used for liver tissue construction provide an ideal 3-D microenvironment for reorganization of primary or transformed hepatocytes to form uniform size cell spheroids. The ICC scaffold geometry supports intense cell-to-cell contacts, and hydrogel matrix minimizes cell-to-matrix interactions. As a result, cells seeded on an ICC scaffold form spheroids in a relatively short time, which significantly improves hepatocyte viability and functionality. Furthermore, the spherical shape of pores constrains the size of cell spheroids. Cell spheroids that are fairly uniform in size are formed over the ICC scaffold.

The template of an ICC scaffold is prepared with soda lime glass beads which have diameter less than 200µm. Highly ordered and packed colloidal crystals are made following the previously mentioned method. Its dimensions exactly fit the size of a single well of the standard 96 well-plate. Acrylamide hydrogel precursor solution is infiltrated into the colloidal crystals and polymerized followed by adding an initiator. Glass beads are dissolved by 5% hydrogen fluoride (HF) solution. To completely diffuse away HF from a hydrogel matrix, ICC scaffolds are thoroughly washed with 2 DI-water (PH=2) or PBS buffer solution several times. Then the ICC scaffolds are freeze dried to evaporate remaining HF. Dried ICC scaffolds are rehydrated with PBS buffer solution and sterilized by 70% ethanol followed by 3 hours UV treatment.

Transformed human hepatoblastoma cell line HepG2, or other human or mouse primary hepatocytes will be used. The media composition will be William's E medium supplemented with 10% FBS, 0.5µg/ml insulin, 10⁻⁷M dexamethasone, and 1% antibiotics. Approximately 1 million trypsinzed cells will be seeded on one ICC scaffold. To improve cell seeding efficiency, cells seeding will be assisted by centrifugation. An ICC scaffold will be put on a 500µL capacity centrifugal filter device which has 0.65µm pore size. One million cells in a 500µL suspension will be seeded on top of the scaffold, and it will be centrifuged at 1000 rpm for 5min. Cell seeded ICC scaffolds will be transferred to a 96 well-plate.

The cell spheroids are normally formed within 3 days. On day 1, 3, 5 and 7, the medium and scaffolds samples will be collected. The viability and morphological change of cells on scaffolds will be examined under a confocal microscope utilizing a standard live-dead cell assay kit and scanning electron microscope, respectively. Albumin secretion will be analyzed using an ELISA based assay with purified albumin standard and albumin fluorescence reagent. The cells will be treated with 1mM NH₄Cl for 4 hours and the produced urea will be measured using an ELISA with dehydrogenase assay kit. At the end of culture, an MTT assay or dsDNA quantification will be performed. These results will be used to normalize ELISA data depending on the actual cell numbers residing in ICC scaffolds.

Once the cell viability has been maintained and basic functionality of hepatocytes confirmed, its ability to produce cytochrome P450 (CYP) will be tested using a training set of chemical compounds. Five distinct inducers such as 3-methylcholanthrene, phenobarbital, rifampin, isoniazid, and efavirenz, will be added to the hepatocyte model system and the released inducer-specific CYP isozymes will be characterized by isozyme identification reagents. Three different concentrations of inducers (10µM, 5µM, and 2.5µM) will be added in culture medium and incubated for 48h with a replacement of medium at 24h. The released CYP isozymes will be characterized using an ELISA with identification reagent. After confirming, CYP isozymes secretion potential, biotransformation capability and standardization of the model system activity will be validated by applying a training set of fully characterized CYP inhibitors/inducers *in-vivo* experiments will be used. The combination of each CYP isozyme specific substrate/inducers or substrate/inhibitors will be added in the hepatocytes model system. Enzyme activities corresponding to the concentration of inducers and inhibitors will be quantitatively characterized by measuring fluorescent intensity. Vivid® CYP substrates will release fluorescent light after consumed by CYP isozymes.

While the present disclosure teaches the principles of the present disclosure, with examples provided for the purpose of illustration, it will be appreciated by one of ordinary skill in the art from reading this disclosure that some changes in form and detail can be made without departing from the spirit and scope of the invention.

### Conclusion

To summarize the foregoing, the invention relates to an artificial bone marrow construct comprising a substrate having at least one well; a three dimensional biocompatible polymer matrix comprising a transparent polymer network containing microspherical voids, wherein the microspherical voids are each connected to at least one other void through inter-connecting pores; at least one LBL coating on a surface of at least one of the polymer network, voids and pores, a population of bone marrow cells comprising stem cells and stromal cells; and at least one bioactive agent. An artificial immune network comprising a polymer matrix with a population of immune cells comprising B-cells and T-cells is disclosed. Methods for testing the toxicity of drugs and other agents against bone marrow cells and methods for making universal blood using the artificial bone marrow constructs are also disclosed.

## Claims

1. An artificial bone marrow construct comprising:
a substrate having at least one well;
a three dimensional biocompatible polymer matrix disposed in said at least one well, said polymer matrix comprising a transparent polymer network containing microspherical voids, said microspherical voids each being in communication with at least one other void through inter-connecting pores;
at least one Layer by Layer (LBL) coating on a surface of at least one of said microspherical voids and said inter-connecting pores;
a population of bone marrow cells comprising stem cells and stromal cells, said bone marrow cells being disposed within at least one of said microspherical voids; and
at least one bioactive agent added to said bone marrow cells to induce at least one of cell growth and cell differentiation of at least one of said stem cells and said stromal cells.

2. The artificial bone marrow construct according to claim 1, wherein said LBL coating is a polyelectrolyte comprising poly(diallydimethyl) ammonium chloride, clay, metal oxides, non-metal oxides, poly-lysine, poly acetylamine, collagen, extracellular matrix, nanocolloidal cellulose, cellulose derivatives and carbon.

3. The artificial bone marrow construct according to claim 1 or 2, wherein said LBL coating is poly(diallydimethyl) ammonium chloride and clay.

4. The artificial bone marrow construct according to any of claims 1 to 3, wherein said polymer network comprises a porosity ranging between about 50% to about 90%.

5. The artificial bone marrow construct according to any of claims 1 to 4, said microspherical voids have a diameter ranging from about 10 µm to about 500 µm.

6. The artificial bone marrow construct according to any of claims 1 to 5, said inter-connecting pores in communication with said microspherical void have a diameter ranging from about 5 µm to about 50 µm.

7. The artificial bone marrow construct according to any of claims 1 to 6, further comprising a cell culture medium disposed in said well.

8. The artificial bone marrow construct according to any of claims 1 to 7, said stem cells comprise CD34+ stem cells derived from one or more of cord blood, circulating blood and bone marrow.

9. The artificial bone marrow construct according to any of claims 1 to 8, said stromal cells comprise hematopoietic precursor cells, non-hematopoietic cells and combinations thereof.

10. The artificial bone marrow construct according to any of claims 1 to 9, said stromal cells are positive for at least one of CD105 and CD166.

11. A method for testing the toxicity of an agent towards natural bone marrow, the method comprising:
administering an agent selected from the group consisting of a toxin, a drug, a biologic, a medicament, a cosmetic, and combinations thereof to an artificial bone marrow construct, said bone marrow construct comprising a substrate having at least one well; a three dimensional biocompatible polymer matrix disposed in said at least one well, said polymer matrix comprising a transparent polymer network containing microspherical voids, said microspherical voids each being connected to at least one other void through inter-connecting pores; at least one LBL coating on a surface of at least one of said microspherical voids and said inter-connecting pores, said bone marrow construct populated with bone marrow cells; and
evaluating a response of the bone marrow cells to said agent.

12. An artificial immune network comprising:
a three dimensional biocompatible polymer matrix comprising a polymer network containing microspherical voids, wherein said microspherical voids are each connected to at least one other void through inter-connecting pores; and
a population of immune cells comprising B-cells and T-cells disposed within said polymer network.

13. The artificial immune network according to claim 12, wherein said polymer network comprises a transparent hydrogel, polystyrene, a collagen gel, a fibrin gel, poly(lactic acid), poly(glycolic acid), polypeptides, bioglasses, an inorganic gel, and co-polymers and mixtures thereof.

14. The artificial immune network according to claim 12 or 13, wherein said immune cells further comprise hematopoietic stem cells, stromal cells, follicular dendritic cells, dendritic cells, natural killer cells, macrophages, monocytes, neutrophils, basophils, mast cells, eosinophils and antibody producing plasma cells.

15. The artificial immune network according to claim 14, wherein at least a portion of said antibody producing plasma cells produce antibody directed to an antigen selected from the group consisting of a bacterial antigen, a viral antigen, a fungal antigen and a tumor antigen.

16. The artificial immune network according to any of claims 12 to 15, further comprising a bioactive agent, said bioactive agent is at least one of IL-2, IL-7, Flt3 ligand, stem cell factor-1, BMP-4, IL-3, soluble CD40L, IL-4, IL-5, IL-6, IL-10 and agonist anti CD40 mAb.

17. A method of making universal blood, the method comprising the steps:
(a) providing an artificial bone marrow construct comprising a three dimensional biocompatible polymer matrix, said polymer matrix comprising a transparent polymer network containing microspherical voids, wherein said microspherical voids are each connected to at least one other void through inter-connecting pores;
(b) culturing hematopoietic stem cells in the presence of a tissue culture medium comprising a first biological active agent in an amount sufficient to induce the differentiation of hematopoietic stem cells into red blood cell progenitor cells in said artificial bone marrow construct;
(c) adding a second biological active agent to said artificial bone marrow construct, wherein said second biological active agent is in an amount sufficient to increase the numbers of said red blood cell progenitor cells in said artificial bone marrow construct;
(d) adding a third biological active agent to said red blood cell progenitor cells to differentiate said red blood cell progenitor cells to form mature red blood cells;
(e) collecting said mature red blood cells from said artificial bone marrow construct aseptically; and
(f) storing said mature red blood cells in a sterile container for use.

18. The method according to claim 17, wherein the first biological active agent comprises: granulocyte macrophage colony stimulating factor (GM-CSF) and interleukin-3 (IL-3).

19. The method according to claim 17 or 18, wherein said second biological active agent comprises: stem cell factor (SCF), granulocyte macrophage colony stimulating factor (GM-CSF), transforming growth factor type (TGF- α), erythropoietin, and steroid hormones.

20. The method according to any of claims 17 to 19, wherein said third biologically active agent comprises erythropoietin and insulin.

21. The method according to any of claims 17 to 20, further comprising removing AB antigens on the surface of said mature red blood cells.
